# EUROPÄISCHE PATENTSCHRIFT

(11) **EP 2 937 619 B1**
(45) Veröffentlichungstag und Bekanntmachung des Hinweises auf die Patenterteilung: **15.03.2017**
(21) Anmeldenummer: 14001481.2
(22) Anmeldetag: 24.04.2014
(51) Int. Cl.: F16M 11/08, F16M 11/20, F16M 13/02, F16C 11/10

(54) **Drehbare Verbindung mit Drehwinkelbegrenzung**
Rotatable connection with limitation of the rotational angle
Liaison rotative doté d'une limitation d'angle de rotation

(43) Veröffentlichungstag der Anmeldung: 28.10.2015
(73) Patentinhaber: Ondal Medical Systems GmbH, 36088 Hünfeld (DE)
(72) Erfinder: Oginski, Stefan, 36041 Fulda (DE); Bauditz, Ronny, 98528 Suhl (DE); Göbel, Andreas, 36132 Eiterfeld (DE); Euler, Annika, 36088 Hünfeld (DE)
(74) Vertreter: Graf von Stosch, Andreas

(56) Entgegenhaltungen:
- EP-A1- 0 392 303
- EP-B1- 2 325 541
- DE-A1-102008 011 129
- DE-C1- 4 306 802
- US-A1- 2011 314 637

## Beschreibung

Die vorliegende Erfindung betrifft eine drehbare Verbindung für eine Stativvorrichtung zur Anordnung in einem Operationssaal, die einen anpassbaren Anschlagmechanismus aufweist, der zwischen einer Spindel und einer relativ zur Spindel verdrehbar um eine Drehachse gelagerten Buchse anordenbar ist und eingerichtet ist, mindestens zwei unterschiedliche relative Drehwinkel der Spindel relativ zur Buchse oder mindestens zwei unterschiedliche Drehbereiche festzulegen, wobei der anpassbare Anschlagmechanismus aufweist: einen ersten Teil, insbesondere in Form eines Rings, welcher verdrehsicher an der Spindel lagerbar ist und mindestens einen Anschlag aufweist; und einen zweiten Teil, welcher verdrehsicher an der Buchse anordenbar bzw. vorgesehen ist; wobei der erste Teil relativ zum zweiten Teil verdrehbar gelagert ist. Die vorliegende Erfindung betrifft insbesondere eine drehbare Verbindung mit einzelnen Merkmalen des Anspruchs 1 sowie ein Tragsystem bzw. eine Stativvorrichtung mit einzelnen Merkmalen des entsprechenden weiteren unabhängigen Anspruchs sowie ein Verfahren zum Einstellen des anpassbaren Anschlagmechanismus mit einzelnen Merkmalen des entsprechenden unabhängigen Verfahrensanspruchs.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger auf, mittels welchen eine daran befestigte medizintechnische Einrichtung bewegt und positioniert werden kann, z.B. in einem Operationssaal, insbesondere auch auf einer Intensivstation. An den Stativen sind häufig Versorgungseinheiten montiert, mit denen medizinisch-elektrische Endgeräte mit den z.B. während einer Operation benötigten Medien versorgt werden können. Die Träger definieren dabei einen Aktionsradius der medizintechnischen Einrichtung, in welchem die medizintechnische Einrichtung positioniert ist. Die Träger können meist zumindest um mindestens eine drehbare Verbindung, insbesondere ein Drehgelenk verdreht werden. Wahlweise sind die Träger auch höhenverstellbar und/oder um eine zumindest annähernd horizontal ausgerichtete Achse in der Höhe verschwenkbar angeordnet.

Eine Drehbewegung einzelner Träger, sei es eine absolute Drehbewegung oder eine Drehbewegung relativ zu einem anderen Träger, soll in vielen Fällen auf einen vorgegebenen Winkel begrenzt werden. Hierdurch kann z.B. vermieden werden, dass ein Träger um mehr als 360° gegenüber einem anderen Träger verdreht wird und dadurch im Träger geführte Leitungen verdrillt, eingequetscht oder sogar abgerissen werden. Eine Drehwinkelbegrenzung kann z.B. in Form eines Anschlags bereitgestellt werden, an welchem ein Träger bei einem bestimmten Verdrehwinkel, z.B. 300°, anschlägt. Der Anschlag kann dabei z.B. fest am Träger montiert sein, insbesondere in Form eines in radialer Richtung eingebrachten Sicherungsbolzens. Der Anschlag gibt dabei einen vordefinierten Drehwinkel vor. Eine solche Drehwinkelbegrenzung kann zwar sicherstellen, dass ein maximaler Drehwinkel nicht überschritten wird, hat aber meist auch den Nachteil, dass die Bewegungsfreiheit des Stativs eingeschränkt wird, also dass sich z.B. eine Versorgungseinheit des Stativs nicht mehr in beliebigen Positionen anordnen lässt. Der Aktionsradius des Stativs wird eingeschränkt, insbesondere ohne Berücksichtigung einer bestimmten Raumsituation. Deshalb muss in jedem Einzelfall erwogen werden, durch welchen Anschlag die Drehwinkelbegrenzung definiert werden kann oder soll. Die richtige Auslegung der Drehwinkelbegrenzung, insbesondere eine adäquate Positionierung des Anschlags, kann jedoch bereits bei der Herstellung eines jeweiligen Stativs Schwierigkeiten mit sich bringen, insbesondere dann, wenn noch nicht geklärt ist, an welchem Ort das Stativ jeweils eingesetzt werden soll. Daher sind Drehwinkelbegrenzungen praktisch, mittels welchen ein Drehwinkel oder eine Drehposition nachträglich angepasst werden kann.

Eine Vorrichtung mit einem einstellbaren Drehwinkel gemäß dem Oberbegriff des Anspruchs 1 ist aus der EP 2 325 541 B1 bekannt.

In der EP 2 325 541 B1 wird ein zweiteiliger anpassbarer Anschlagmechanismus beschrieben, bei welchem ein ringförmiges Teil selektiv außen um einen Umfang eines ersten Trägers bzw. eines Gelenks des ersten Trägers herum positioniert werden kann, und das ringförmige Teil weist eine Vielzahl von stirnseitig angeordneten Ausnehmungen oder Vorsprünge auf, mittels welchen es relativ zum ersten Träger auf einfache Weise in unterschiedlichen Drehwinkelpositionen anordenbar ist. Am ringförmigen Teil ist ferner ein Anschlag angeordnet, an welchem ein zweiter Träger anschlagen kann. Mittels des ringförmigen Teils kann ein Drehwinkel der beiden Träger relativ zueinander eingestellt werden. Der Anschlagmechanismus ist dabei innerhalb von einem Bund des zweiten Trägers angeordnet.

Das ringförmige Teil kann durch Eingriff eines Werkzeugs in eine an einer Außenmantelfläche des ringförmigen Teils umlaufende Nut angehoben werden, um das ringförmige Teil relativ zum ersten Träger in einer gewünschten Drehwinkelposition zu positionieren. Ferner ist ein weiteres ringförmiges Teil am ersten Träger vorgesehen, welches relativ zum ringförmigen Teil positioniert werden kann. Die beiden ringförmigen Teile sind innerhalb vom Bund angeordnet und werden vom Bund radial außen umschlossen und abgedeckt. Im Bund ist ein in radialer Richtung eingebrachter Sicherungsbolzen angeordnet, welcher in einen zwischen den beiden ringförmigen Teilen gebildeten Zwischenraum eingereift. Durch die relative Drehposition des ersten Teils relativ zum zweiten Teil wird die Ausdehnung des Zwischenraums in Umfangsrichtung definiert. Über die Ausdehnung des Zwischenraums in Umfangsrichtung kann der Winkelbereich definiert werden, in welchem die beiden Träger relativ zueinander verdreht werden können. Der Anschlagmechanismus ist dabei im Wesentlichen am ersten Träger angeordnet und wirkt über den radial eingebrachten Sicherungsbolzen mit dem zweiten Träger zusammen.

In der DE 38 08 327 A1 wird ein Stoppmechanismus beschrieben, bei welchem ein Gewindebolzen in radialer Richtung in einer Gewindebohrung verlagert werden kann, um unterschiedliche Drehwinkelpositionen einzustellen.

Es ist eine Aufgabe der vorliegenden Erfindung, eine drehbare Verbindung bereitzustellen, mittels welcher ein Drehwinkel bzw. Dreh(winkel)bereich auf einfache Weise einstellbar ist. Insbesondere besteht die Aufgabe auch darin, eine Stativvorrichtung mit einer Drehwinkelbegrenzung bereitzustellen, bei welcher einzelne Träger der Stativvorrichtung dank einer leicht (insbesondere manuell) einstellbaren drehbaren Verbindung auf flexible Weise in einem Operationssaal positioniert werden können.

Diese Aufgabe wird gelöst durch eine drehbare Verbindung für eine Stativvorrichtung zur Anordnung in einem Operationssaal, umfassend einen anpassbaren Anschlagmechanismus, der zwischen einem ersten Verbindungsbauteil (insbesondere einem Verbindungsbauteil der drehbaren Verbindung) und einem relativ zum ersten Verbindungsbauteil verdrehbar um eine Drehachse gelagerten zweiten Verbindungsbauteil (insbesondere einem Verbindungsbauteil der drehbaren Verbindung) anordenbar ist und eingerichtet ist, mindestens zwei unterschiedliche relative Drehwinkel der Verbindungsbauteile relativ zueinander oder mindestens zwei unterschiedliche Drehbereiche festzulegen, wobei der anpassbare Anschlagmechanismus aufweist:
- einen ersten Teil, welcher verdrehsicher an dem ersten Verbindungsbauteil lagerbar ist und mindestens einen Anschlag aufweist;
- einen zweiten Teil, welcher verdrehsicher am zweiten Verbindungsbauteil vorgesehen bzw. anordenbar ist;
wobei der erste Teil relativ zum zweiten Teil verdrehbar gelagert ist;
wobei der anpassbare Anschlagmechanismus mindestens eine oder zwei Anschlageinrichtungen mit jeweils einem Gegenanschlag aufweist, welche axial (also in Bezug auf die axiale Richtung der Drehachse) zwischen den beiden Teilen angeordnet ist/sind und dadurch mit den beiden Teilen zusammenwirken können, wobei der jeweilige Gegenanschlag mit dem Anschlag korrespondiert, und wobei die mindestens eine oder zwei Anschlageinrichtungen eingerichtet ist/sind, mittels des jeweiligen Gegenanschlags die unterschiedlichen relativen Drehwinkel oder Drehbereiche festzulegen. Hierdurch kann eine auf einfache Weise einstellbare drehbare Verbindung bereitgestellt werden. Das Einstellen kann z.B. durch axiales Verlagern und Verdrehen der mindestens einen Anschlageinrichtung erfolgen, insbesondere auf manuelle Weise. Die mindestens eine separate Anschlageinrichtung kann in unterschiedlichen Verdreh-Positionen zwischen den beiden Teilen angeordnet werden. Ein in radialer Richtung angeordneter Stift oder Sicherungsbolzen ist nicht erforderlich. Vielmehr können die Komponenten in axialer Richtung zueinander positioniert werden. Der oder die Gegenanschläge können an der mindestens einen Anschlageinrichtung in vorgegebenen Positionen befestigt sein. Hierdurch ist es auch möglich, unterschiedliche Drehbereiche festzulegen, also z.B. einen Drehbereich von Kompass-Nord (also der geografischen Nord-Richtung) um z.B. 300° im Uhrzeigersinn und entgegen dem Uhrzeigersinn, oder einen Drehbereich ausgehend von Kompass-Ost um 330°, oder einen Drehbereich von Kompass-Nord um 270°. Dadurch kann der Aktionsradius z.B. einer Stativvorrichtung auch in Bezug auf eine Anordnung in Wandnähe oder in einer Ecke angepasst und eingestellt werden. Der (absolute/maximale) Betrag des Drehwinkels kann durch die geometrische Ausführung des Anschlagmechanismus, insbesondere der Anschlageinrichtung, fest vorgegeben werden oder auch eingestellt werden, insbesondere durch Verlagern einer mindestens einen Anschlageinrichtung relativ zur anderen Anschlageinrichtung. Der Startpunkt der Drehbewegung ist einstellbar, indem eine einzelne Anschlageinrichtung versetzt wird oder indem beide Anschlageinrichtungen zusammen in gleichem Maße versetzt werden.

Durch eine Anordnung des Anschlagmechanismus sowohl am ersten Verbindungsbauteil als auch am zweiten Verbindungsbauteil kann mindestens eine Anschlageinrichtung vorgesehen werden, die (jeweils) auf einfache Weise zwischen den beiden Verbindungsbauteilen angeordnet und umpositioniert werden kann, insbesondere durch axiale Verlagerung des ersten Teils relativ zum zweiten Teil.

Mittels einer Anschlageinrichtung, die einen oder mehrere Gegenanschläge aufweist, kann die Anzahl der Komponenten gering gehalten werden. Bevorzugt ist der gesamte Anschlagmechanismus nur aus drei Komponenten aufgebaut, insbesondere dem ersten Teil, dem zweiten Teil und der Anschlageinrichtung.

Die als verdrehsichere Anordnung beschriebene Verbindung oder Lagerung des zweiten Teils zum bzw. am Verbindungsbauteil kann dabei z.B. durch eine Nut-Feder-Verbindung bereitgestellt werden, also eine Verbindung, die nur eine einzige Relativposition der beiden Bauteile zueinander definiert. Eine verdrehsichere Anordnung, Verbindung oder Lagerung kann dabei auch eine Anordnung umfassen, bei welcher der zweite Teil (einstückig) als ein integraler Teil des Verbindungsbauteils ausgebildet ist. Insbesondere kann der zweite Teil in ein als Buchse ausgebildetes zweites Verbindungsbauteil integriert sein.

Bevorzugt ist der erste Teil zwar verdrehsicher am ersten Verbindungsbauteil gelagert, dies jedoch bevorzugt nur in Hinblick auf eine Drehbewegung. Mit anderen Worten: die verdrehsichere Anordnung bringt nicht notwendigerweise eine vordefinierte Axialposition mit sich. Vielmehr ist der erste Teil in axialer Richtung bevorzugt am zweiten Verbindungsbauteil gelagert, insbesondere mittels der mindestens einen Anschlageinrichtung und/oder dem zweiten Teil. Dabei kann das erste Verbindungsbauteil in axialer Richtung bevorzugt am zweiten Verbindungsbauteil axial positioniert werden, oder umgekehrt, z.B. mittels eines Wellensicherungsrings.

Als "drehbare Verbindung" ist dabei bevorzugt eine Anordnung zu verstehen, mittels welcher eine Verdrehung zweier Bauteile zueinander um einen vorgebbaren Winkel sichergestellt werden kann. Die drehbare Verbindung ist z.B. eine Verbindung zwischen einer Buchse und einer Spindel, wobei die drehbare Verbindung nicht notwendigerweise die Buchse und Spindel umfasst, sondern z.B. nur die daran vorgesehenen Lager oder Lagerflächen dafür. Die drehbare Verbindung weist bevorzugt mindestens ein Drehgelenk auf oder bildet einen Teil des Drehgelenks. Als Drehgelenk ist dabei bevorzugt ein Gelenk zu verstehen, welches zumindest eine Drehung um eine oder mehrere Drehachsen ermöglicht, wobei auch ein translatorischer Freiheitsgrad verwirklicht sein kann. Das Drehgelenk ist bevorzugt an der Schnittstelle zwischen zwei einzelnen Trägern angeordnet, kann jedoch auch einen einzelnen Träger in mehrere Abschnitte untergliedern. Das Drehgelenk kann z.B. an der Schnittstelle zwischen einer Spindel und einer Buchse vorgesehen sein.

Als "Stativvorrichtung" ist dabei bevorzugt eine Vorrichtung zum Halten, ortsfesten Anordnen und/oder Verlagern mindestens einer medizintechnische Einrichtung zu verstehen, die an einer Wand (in einem Wandlager) oder einer Decke oder auch am Boden eines Operationssaals oder irgendeines anderen Raumes für medizinische Zwecke fest montiert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Decke oder Wand des Operationssaals angeordneten Befestigungspunkt bzw. Montagepunkt. Die Stativvorrichtung kann als an einer Decke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Decke montierte Zentralachse ausgebildet sein und eine Mehrzahl von Tragsystemen mit jeweils mindestens einem Träger aufweisen, an welchem z.B. ein Monitor oder eine Leuchte gelagert ist. Die Stativvorrichtung muss jedoch nicht notwendigerweise fest an einer Wand montiert sein, sondern kann auch auf einem fahrbaren Unterbau montiert sein. Der fahrbare Unterbau kann z.B. mittels Bremsen ortsfest im Raum positioniert werden. Auch in diesem Fall ist ein anpassbarer Anschlagmechanismus zweckdienlich.

Als "anpassbarer Anschlagmechanismus" ist dabei bevorzugt jede Einrichtung zu verstehen, welche einen Drehwinkel und/oder Drehbereich eines Trägers begrenzen kann, insbesondere relativ zu einem weiteren Träger oder relativ zu einer fest im Raum positionierten (fiktiven) Drehachse, z.B. eine durch einen fest angeordneten Befestigungspunkt an einer Wand eines Raumes verlaufende Drehachse. Bevorzugt weist der anpassbare Anschlagmechanismus zumindest auch eine formschlüssig ausgebildete Verbindung auf oder ist für Formschluss ausgebildet. Der anpassbare Anschlagmechanismus kann zusätzlich auch kraftschlüssig wirken.

Als "Drehbereich" ist dabei bevorzugt ein Winkelbereich zu verstehen, in welchem ein Träger relativ zu einem weiteren Träger oder zu einer Wand verdreht werden kann. Der Winkelbereich kann z.B. zwischen 90° und 330° betragen. Der Winkelbereich kann konstant groß sein, aber z.B. in Bezug auf unterschiedliche Umfangspositionen festgelegt werden, also z.B. von 0° bis 300° in Bezug auf eine Nord-Richtung, oder von 30° bis 330° in Bezug auf die Ost-Richtung. Der Drehbereich kann durch unterschiedliche Drehwinkelpositionen definiert werden, in welchen Gegenanschläge der Anschlageinrichtung(en) anordenbar sind.

Als "erster Teil" ist dabei bevorzugt ein Teil zu verstehen, welcher irgendwie verdrehsicher an die Drehbewegung des ersten Verbindungsbauteils (z.B. einer Spindel) gekoppelt ist und bevorzugt formschlüssig mit dem ersten Verbindungsbauteil zusammenwirkt. Der erste Teil ist dabei bevorzugt in axialer Richtung relativ zum ersten Verbindungsbauteil verlagerbar, insbesondere in Richtung der Drehachse. In Umfangsrichtung ist eine relative Verlagerung zueinander blockiert oder zumindest ab einem gewissen Drehwinkel blockierbar. Der erste Teil kann z.B. ringförmig ausgebildet sein und dann als Anschlagring bezeichnet werden, welcher mindestens einen Anschlag definiert. Als Anschlag ist dabei bevorzugt irgendein in axialer Richtung hervorstehender oder eine Überlappung sicherstellender Vorsprung oder Absatz zu verstehen.

Als "zweiter Teil" ist dabei bevorzugt ein Teil zu verstehen, welcher verdrehsicher an die Drehbewegung des zweiten Verbindungsbauteils (z.B. einer Buchse) gekoppelt ist und z.B. formschlüssig mit dem zweiten Verbindungsbauteil zusammenwirkt, insbesondere drehsynchron. Mit anderen Worten ist der zweite Teil derart am zweiten Verbindungsbauteil vorgesehen, dass der zweite Teil und das zweite Verbindungsbauteil jedenfalls dieselbe Drehbewegung ausführen. Die Position des zweiten Teils relativ zum zweiten Verbindungsbauteil ist dann vordefiniert und auch nicht veränderbar. Der zweite Teil kann durch das zweite Verbindungsbauteil gebildet sein, z.B. angegossen sein. Bevorzugt ist der zweite Teil ortsfest am zweiten Verbindungsbauteil vorgesehen, also auch axialfest, d.h. in axialer Richtung relativ zum zweiten Verbindungsbauteil nicht verlagerbar. Der zweite Teil ist bevorzugt nur mit dem zweiten Verbindungsbauteil verbunden oder dadurch gebildet und ist vom ersten Verbindungsbauteil entkoppelt und wirkt nur mittels der mindestens einen Anschlageinrichtung und des ersten Teils indirekt mit dem ersten Verbindungsbauteil zusammen. Der zweite Teil kann z.B. ringförmig ausgebildet sein und kann mindestens eine formschlüssige Kontur in Form einer Verzahnung aufweisen, z.B. eine Sägezahnkontur, insbesondere an der Schnittstelle zur Anschlageinrichtung. Der zweite Teil kann dann als Zahnkranz bezeichnet werden. Bevorzugt sind am zweiten Teil keine einen Drehbereich definierenden Anschläge bzw. Gegenanschläge angeordnet. Derartige Anschläge sind nicht erforderlich, insbesondere weil eine relative Drehbewegung zwischen dem zweiten Teil und der mindestens einen Anschlageinrichtung nicht stattfinden muss bzw. soll. Bevorzugt ist der zweite Teil eingerichtet, die mindestens eine Anschlageinrichtung drehfest in einer einstellbaren Drehposition am zweiten Verbindungsbauteil zu lagern, so dass ein Anschlag des ersten Teils an der mindestens einen Anschlageinrichtung anschlagen kann, um eine entsprechend hervorgerufene Reaktionskraft von der mindestens einen Anschlageinrichtung auf den zweiten Teil zu übertragen. Mit anderen Worten: der erste Teil ist bevorzugt nur mittelbar mit dem zweiten Teil in Verbindung, insbesondere über die Anschlageinrichtung.

Als "Anschlageinrichtung" ist dabei bevorzugt ein Teil zu verstehen, welcher dazu eingerichtet ist, einen Gegenanschlag in einer relativ zu einem der Verbindungsbauteile, insbesondere relativ zum zweiten Verbindungsbauteil, ortsfesten Position bereitzustellen, wobei eine in Umfangsrichtung auf die Anschlageinrichtung ausgeübt (Verdreh-)Kraft, also ein Drehmoment, über den Gegenanschlag zwischen den Verbindungsbauteilen übertragen werden kann. Die Anschlageinrichtung ist bevorzugt eingerichtet, ein direktes Zusammenwirken zwischen dem ersten und zweiten Teil zu verhindern. Die mindestens eine Anschlageinrichtung ist bevorzugt zwischen dem ersten und zweiten Teil zwischengelagert und eingerichtet, ein Drehmoment zwischen dem ersten Teil und dem zweiten Teil zu übertragen. Bevorzugt erstreckt sich die Anschlageinrichtung zumindest abschnittsweise um die Drehachse herum, wobei die Anschlageinrichtung bevorzugt ringförmig ausgebildet ist und umlaufend um die Drehachse vorgesehen ist. Die Anschlageinrichtung kann dann z.B. als Stellring beschrieben werden. Als Gegenanschlag ist dabei bevorzugt irgendein Vorsprung, Absatz oder eine hervorstehende Nase zu verstehen.

Als eine Anordnung "axial zwischen" dem ersten und zweiten Teil ist dabei bevorzugt eine Anordnung zu verstehen, bei welcher der erste und zweite Teil nicht direkt miteinander gekuppelt sind, sondern nur indirekt mittels der Anschlageinrichtung. Eine Anordnung "axial zwischen" bedeutet bevorzugt, dass der erste Teil in axialer Richtung nicht in den zweiten Teil eingreifen muss, sondern dass ein Eingriff oder Zusammenwirken des ersten Teils mit dem zweiten Teil (allein) mittels der mindestens einen Anschlageinrichtung sichergestellt werden kann.

Als "Drehwinkelposition" ist dabei bevorzugt eine relative Verdrehposition einer Anschlageinrichtung in Bezug auf eines der Verbindungsbauteile, insbesondere in Bezug auf das zweite Verbindungsbauteil zu verstehen. Die Drehwinkelposition kann dabei durch die Relativposition eines Gegenanschlags definiert werden. Die Drehwinkelposition kann auch in Bezug auf einen absoluten (Horizontal-)Winkel beschrieben werden, z.B. um eine (fiktive) vertikal ausgerichtete Drehachse.

Bevorzugt ist die mindestens eine Anschlageinrichtung eingerichtet, eine in Umfangsrichtung wirkende Drehkraft, die auf den Anschlag bzw. den Gegenanschlag ausgeübt wird, zwischen dem ersten und zweiten Teil zu übertragen, also vom ersten Teil auf den zweiten Teil und/oder vom zweiten Teil auf den ersten Teil. Mit anderen Worten: die mindestens eine Anschlageinrichtung ist eingerichtet, die beiden Teile aneinander zu kuppeln, insbesondere auch um einen bestimmten Drehwinkelbereich der Teile relativ zueinander zu definieren.

Gemäß einem Ausführungsbeispiel ist mindestens eine der Anschlageinrichtungen verdrehsicher zu einem der beiden Teile, insbesondere zum zweiten Teil, an einem der beiden Teile in mindestens zwei unterschiedlichen Drehwinkelpositionen positionierbar. Hierdurch kann ein Startpunkt bzw. Ausgangspunkt eines bestimmten Drehwinkelbereichs eingestellt werden. Bevorzugt ist der Gegenanschlag ortsfest an der mindestens einen Anschlageinrichtung positioniert. Der Gegenanschlag kann integral an der mindestens einen Anschlageinrichtung vorgesehen sein, d.h., die Anschlageinrichtung bildet mit dem Gegenanschlag ein einstückiges Teil. Hierdurch kann eine geringe Anzahl von Teilen oder Komponenten eingehalten werden. Insbesondere kann vermieden werden, dass kleine Stellschrauben oder Radialbolzen verloren gehen. Wahlweise kann der Gegenanschlag oder mindestens ein Gegenanschlag von einer Vielzahl von Gegenanschlägen auch an der mindestens einen Anschlageinrichtung befestigt sein, z.B. mittels einer Verschraubung in radialer oder axialer Richtung. Dies erleichtert z.B. das Einstellen eines bestimmten Drehwinkels.

Bevorzugt ist der erste Teil in axialer Richtung entlang der Drehachse verlagerbar angeordnet. Hierdurch kann die mindestens eine Anschlageinrichtung zusammen mit dem ersten Teil auf einfache Weise in axialer Richtung verschoben werden, um den Drehbereich bzw. Drehwinkel einzustellen. Es ist nicht erforderlich, irgendeinen in radialer Richtung eingreifenden Bolzen oder einen den Bolzen aufnehmenden Bund zu entfernen, um die beiden Teile relativ zueinander in axialer Richtung zu verlagern. Hierbei kann der erste Teil über eine Zentrierung an einer Innenmantelfläche am ersten Verbindungsbauteil geführt werden. Wahlweise kann die Anschlageinrichtung ohne den ersten Teil angehoben werden, insbesondere indem zuvor eine Sicherungseinrichtung (z.B. Wellensicherungsring) entfernt wird, welches oberhalb vom ersten Teil anordenbar ist und eine axiale Verschiebung verhindern kann.

Bevorzugt ist die mindestens eine der mindestens einen Anschlageinrichtung in axialer Richtung entlang der Drehachse verlagerbar angeordnet, insbesondere zusammen mit dem ersten Teil. Hierdurch kann das Einstellen der drehbaren Verbindung auf einfache Weise erfolgen. Dabei braucht z.B. nur die mindestens eine Anschlageinrichtung ergriffen zu werden, und der erste Teil kann zusammen mit der mindestens einen Anschlageinrichtung axial verschoben werden, insbesondere nach oben entgegen einer Gravitationskraft.

Bevorzugt sind der erste Teil und die mindestens eine oder zwei Anschlageinrichtungen in axialer Richtung ineinander gesteckt oder zumindest einander axial überlappend angeordnet.

Bevorzugt sind der erste Teil und der zweite Teil und die mindestens eine Anschlageinrichtung in axialer Richtung in Reihe hintereinander angeordnet. Hierdurch kann die drehbare Verbindung, insbesondere der Ausgangspunkt bzw. Startpunkt des Drehwinkelbereichs, auf einfache Weise eingestellt werden, insbesondere nach einem axialen voneinander weg Schieben des ersten Teils und der Anschlageinrichtung.

Durch axiale Verlagerung der Teile bzw. der mindestens einen Anschlageinrichtung zueinander kann eine Einstellung des Drehbereichs auf einfache Weise vorgenommen werden. Auch kann auf einfache Weise ein Dämpfungselement zwischen den Teilen bzw. einem der Teile und der mindestens einen Anschlageinrichtung vorgesehen werden. Die Anordnung in Reihe hintereinander ermöglicht auch eine einfache Montage. Als eine Anordnung in Reihe hintereinander ist dabei eine Anordnung zu verstehen, bei welcher (abgesehen von einem möglicherweise dazwischen angeordneten Dämpfungselement) der erste Teil an der/einer Anschlageinrichtung zur Anlage kommt, und bei welcher die oder eine weitere Anschlageinrichtung am zweiten Teil zur Anlage kommt.

Bevorzugt ist die mindestens eine Anschlageinrichtung in axialer Richtung zwischen den beiden Teilen angeordnet und überlappt in axialer Richtung den zweiten Teil im Bereich einer formschlüssigen Kontur und überlappt in axialer Richtung den ersten Teil im Bereich des Anschlags. Indem der erste Teil und die mindestens eine Anschlageinrichtung in axialer Richtung überlappend angeordnet werden, kann der Anschlagmechanismus in der Form eines einfach aufgebauten Stecksystems bereitgestellt werden. Auch kann eine gute Stabilität der Anordnung sichergestellt werden, insbesondere da sich der erste Teil und die mindestens eine Anschlageinrichtung gegen ein Verkippen stabilisieren können, spezielle über die Innen- und/oder Außenmantelfläche der Anschlageinrichtung. Bevorzugt ist der erste Teil derart dimensioniert und geometrisch ausgebildet, dass der erste Teil, insbesondere eine umlaufende Innen- oder Außenwandung, zumindest teilweise innen oder außen um die mindestens eine Anschlageinrichtung anordenbar ist. Diese Ausgestaltung ermöglicht eine Anordnung des jeweiligen Gegenanschlags in einer vom ersten Teil gebildeten Ringkavität, wodurch sich die Komponenten gegenseitig sichern, stabilisieren und/oder zentrieren können.

Bevorzugt erstreckt sich der erste Teil und/oder der zweite Teil zumindest abschnittsweise um die Drehachse herum, wobei der erste Teil und/oder der zweite Teil bevorzugt ringförmig ausgebildet ist und umlaufend um die Drehachse vorgesehen ist.

Gemäß einer Variante ist der erste Teil ringförmig ausgebildet und weist zwei oder mehr Anschläge auf, die gegenüberliegend zueinander angeordnet sind und in axialer Richtung von einer Scheibe des ersten Teils hervorstehen, insbesondere an einer Außenmantelfläche bzw. Kreisringfläche. Der erste Teil kann dabei einen rotationssymmetrisch ausgebildeten, insbesondere scheibenartigen Bereich aufweisen. Als Scheibe ist dabei bevorzugt ein weitgehend eben ausgebildetes Teil zu verstehen, welches sich im Wesentlichen in einer in radialer Richtung ausgerichteten Ebene erstreckt, mit einer deutlich geringeren Erstreckung in einer axialen Richtung orthogonal zu der Ebene. Eine Ausgestaltung als Scheibe hat den Vorteil, dass eine Gleitfläche auf einfache Weise an einer jeweiligen Stirnfläche der Scheibe bereitgestellt werden kann.

Gemäß einem Ausführungsbeispiel ist der erste Teil eingerichtet, den Anschlag mit mindestens jeweils einem Gegenanschlag von mindestens zwei Anschlageinrichtungen zu kuppeln, insbesondere durch axiales Überlappen in axialer Richtung. Das in Eingriff Bringen eines Anschlags mit mehreren Gegenanschlägen ermöglicht eine hohe Varianz beim Anpassen des Anschlagmechanismus. Bevorzugt kann das Kuppeln oder in Eingriff bringen derart erfolgen, dass der Anschlag entweder mit jedem Gegenanschlag separat in einzelnen Drehwinkelpositionen zusammenwirkt oder gleichzeitig mit mindestens zwei Gegenanschlägen in derselben Drehwinkelposition. Dies ermöglicht eine flexible Auslegung des Anschlagmechanismus, sei es mit nur einer Anschlageinrichtung oder mit zwei oder noch mehr Anschlageinrichtungen. Insbesondere können dadurch sowohl der Drehbereich als auch der Drehwinkel unabhängig voneinander eingestellt bzw. angepasst werden.

Der Anschlag kann dabei integral am ersten Teil vorgesehen sein, d.h., der erste Teil bildet mit dem Anschlag ein einstückiges Teil. Hierdurch kann eine geringe Anzahl von Teilen oder Komponenten eingehalten werden. Insbesondere kann vermieden werden, dass kleine Stellschrauben oder Radialbolzen verloren gehen.

Gemäß einem Ausführungsbeispiel ist der erste Teil ringförmig und eingerichtet, den Gegenanschlag der mindestens einen Anschlageinrichtung in axialer Richtung mittels des Anschlags zu überlappen. Dies liefert den Vorteil eines einfach aufgebauten Anpassmechanismus, der z.B. auf leichte Weise manuell in axialer Richtung steckbar ist.

Gemäß einem Ausführungsbeispiel ist der erste Teil als doppelwandiger Ring bzw. Anschlagring ausgebildet, insbesondere mit U-förmigem Querschnittsprofil, wobei der Anschlag in Form eines sich in radialer Richtung erstreckenden Stegs ausgebildet ist. Hierdurch ist der Anschlagring eingerichtet, den Gegenanschlag der mindestens einen Anschlageinrichtung in axialer Richtung zu umgreifen, also sowohl radial innen als auch radial außen in axialer Richtung zu überlappen. Dabei können auch mehrere Anschlageinrichtungen bzw. Gegenanschläge gleichzeitig umgriffen werden und mit dem Anschlag zusammenwirken. Dabei kann ein Drehimpuls in demselben axialen Abschnitt der drehbaren Verbindung weitergeleitet werden, insbesondere von einer Nut-Feder-Verbindung zwischen dem ersten Verbindungsbauteil und dem ringförmigen ersten Teil über den Anschlag auf den einen oder die mehreren Gegenanschläge. Biegemomente oder Hebelkräfte können daher gering gehalten werden. Auch kann eine in sich geschlossene Bauform ohne irgendwelche hervorstehenden Abschnitte oder Vorsprünge bereitgestellt werden, was die Bedienungssicherheit erhöht (z.B. vermindertes Risiko von Quetschungen oder Einklemmen oder Verkanten). Hierdurch kann auch eine in radialer Richtung besonders schlanke Bauform realisiert werden. Die ringförmige Ausgestaltung des ersten Teils eignet sich insbesondere im Zusammenhang mit einer Deckenbefestigung bei beengten Platzverhältnissen in radialer Richtung. Bevorzugt weist der Anschlagring ein ringförmig umlaufendes U-förmiges Querschnittsprofil mit einer zumindest annähernd planen Oberseite auf, welche die Schenkel des U-Profils miteinander verbindet. Diese Geometrie ermöglicht z.B. auch auf einfache Weise ein formschlüssiges Zusammenwirken des ersten Teils mit einer Sicherungseinrichtung.

Bevorzugt verbindet der Steg eine Innenwand mit einer Außenwand des Rings. Bevorzugt ist der Steg als Schott zwischen der Innenwand mit der Außenwand ausgebildet. Ein Schott hat z.B. auch den Vorteil, dass die Reaktionskräfte auf den Anschlagring weitgehend unabhängig davon sind, ob das Schott mit einem oder mehreren Gegenanschlägen zusammenwirkt. Drehimpulse können sowohl auf die Innenwand als auch auf die Außenwand des Anschlagrings weitergeleitet werden. Biegemomente können gering gehalten werden. Hierdurch kann auch eine besonders robuste Anordnung, insbesondere robuste Ausgestaltung des Anschlags, mit vorteilhaften Eigenschaften in Hinblick auf die Aufnahme und Weiterleitung von Drehimpulsen bereitgestellt werden.

Bevorzugt sind die Innenwand und Außenwand des Anschlagrings zumindest annähernd parallel zueinander angeordnet und erstrecken sich zumindest annähernd in Richtung der Drehachse. Diese Ausgestaltung als Parallel-Doppelring kann ein besonders sicheres Eingreifen der Komponenten ineinander sicherstellen, und ermöglicht auch eine schlanke Bauform. Insbesondere wird das in Eingriff bringen einer Mehrzahl von Anschlageinrichtungen erleichtert.

Bevorzugt ist der Gegenanschlag der mindestens einen Anschlageinrichtung in einer Ringkavität des Anschlagrings gelagert. In der Ringkavität können mehrere Gegenanschläge mehrerer Anschlageinrichtungen zusammen geführt werden und zusammen mit dem Anschlag zusammenwirken. Gleichzeitig kann jede Anschlageinrichtung an einer korrespondierenden Mantelfläche des Anschlagrings entlang gleiten oder entlang geführt werden, was einen Leichtlauf verbessert oder das Risiko eines Verkantens vermindert.

Gemäß einem Ausführungsbeispiel sind der erste Teil und die mindestens eine oder zwei Anschlageinrichtungen und wahlweise auch der zweite Teil zum Einstellen einzelner Drehwinkelpositionen bzw. zum Anpassen des Anschlagmechanismus in axialer Richtung am zweiten Verbindungsbauteil axial anordenbar bzw. axial positionierbar bzw. gelagert. Bevorzugt sind zumindest der erste Teil und die Anschlageinrichtungen in axialer Richtung allein aufgrund der Gewichtskraft axial am zweiten Verbindungsbauteil positioniert. Diese freie Anordnung (ohne zusätzliche Befestigungsmittel) kann einen auf besonders einfache Weise einstellbaren bzw. anpassbaren Anschlagmechanismus bereitstellen.

Gemäß einem Ausführungsbeispiel weist der anpassbare Anschlagmechanismus an vier Schnittstellen, insbesondere axial stirnseitigen Schnittstellen, jeweils eine formschlüssige Kontur auf. Bevorzugt weist eine Komponente des anpassbare Anschlagmechanismus, insbesondere ein unterer Stellring, beidseitig eine formschlüssige Kontur auf. Auf diese Weise kann mit einer geringen Anzahl an Komponenten (speziell drei Komponenten: oberer Stellring, unterer Stellring und zweiter Teil oder Zahnkranz) eine große Variabilität und eine einfache Handhabung sichergestellt werden.

Gemäß einem Ausführungsbeispiel ist die mindestens eine Anschlageinrichtung derart angeordnet, dass eine verdrehsichere Anordnung der mindestens einen Anschlageinrichtung am zweiten Teil (insbesondere ausschließlich) durch eine auf die mindestens eine Anschlageinrichtung wirkende Gewichtskraft bzw. Gravitationskraft sichergestellt ist. Hierbei muss zum Anpassen des Anschlagmechanismus lediglich eine Verlagerung der mindestens einen Anschlageinrichtung entgegen einer auf die mindestens eine Anschlageinrichtung wirkenden Gewichtskraft erfolgen, insbesondere zusammen mit dem ersten Teil. Es ist nicht erforderlich, irgendwelche radial eingebrachten Sicherungsstifte oder -schrauben zu entfernen. Vielmehr kann mittels eines Sicherungsrings eine axiale Sicherung des ersten Teils erfolgen.

Gemäß einem Ausführungsbeispiel weist der zweite Teil eine formschlüssige Kontur zum Festlegen der einzelnen Drehwinkelpositionen auf, insbesondere an einer in die axiale Richtung weisenden Oberseite. Die mindestens eine Anschlageinrichtung oder eine der Anschlageinrichtungen weist eine damit korrespondierende formschlüssige Kontur auf, insbesondere an einer in axialer Richtung zum zweiten Teil oder zweiten Verbindungsbauteil hin weisenden Unterseite. Hierdurch kann eine leicht zugängliche Steckverbindung bereitgestellt werden, mittels welcher der Anschlagmechanismus eingestellt bzw. angepasst werden kann.

Als eine formschlüssige Kontur ist dabei bevorzugt eine Verzahnung bzw. Zahnkontur oder eine Kontur mit regelmäßigen Absätzen oder Vorsprüngen zu verstehen. Dabei ist die Form eines einzelnen Zahns weitgehend beliebig. Bevorzugt weist der einzelne Zahn die Form eines Quaders bzw. im Querschnitt gesehen die Form eines Rechtecks auf. Die formschlüssige Kontur ist nicht notwendigerweise ausschließlich formschlüssig, sondern kann auch kraftschlüssig sein. Die formschlüssige Kontur ist bevorzugt nicht stoffschlüssig, um sicherzustellen, dass der mindestens eine Gegenanschlag reversibel und beliebig häufig in unterschiedlichen Drehwinkelpositionen positionierbar ist.

Bevorzugt ist die formschlüssige Kontur des zweiten Teils in einer axialen Richtung zumindest annähernd parallel zur Drehachse derart zugänglich, dass die entsprechende Anschlageinrichtung mit der korrespondierenden formschlüssigen Kontur in der axialen Richtung auf den zweiten Teil aufsteckbar ist. Dies erleichtert sowohl die Montage als auch das Einstellen.

Bevorzugt sind die formschlüssige Kontur des zweiten Teils und die formschlüssige Kontur der mindestens einen Anschlageinrichtung jeweils als Zahnkranz ausgebildet, wobei Zähne des Zahnkranzes bevorzugt in einer axialen Richtung zumindest annähernd parallel zur Drehachse hervorstehen. Als Zahnkranz ist dabei bevorzugt eine in Bezug auf die Drehachse rotationssymmetrisch ausgebildete Kontur mit einer Vielzahl einzelner Zähne zu verstehen, bei welcher die Zähne in einheitlichem Abstand zueinander angeordnet sind. Die Ausgestaltung als Zahnkranz liefert z.B. den Vorteil kleiner Verstellschritte, denn umso mehr Zähne vorgesehen sind, desto feiner kann der Startpunkt bzw. Ausgangspunkt des Drehwinkelbereichs definiert werden, z.B. in 10°-Schritten.

Gemäß einem speziellen Ausführungsbeispiel bilden der erste Teil und mindestens eine Anschlageinrichtung zusammen ein (Dreh-)Lager, insbesondere ein Gleitlager. Dabei liegt der erste Teil auf der mindestens einen Anschlageinrichtung oder mindestens einer der Anschlageinrichtungen auf, insbesondere auf einem ringförmigen Flächenabschnitt der Anschlageinrichtung, sei es direkt oder sei es mittelbar über eine Sicherungseinrichtung. Hierdurch kann der erste Teil reibungsarm relativ zur mindestens einen Anschlageinrichtung bzw. zum zweiten Teil verlagert werden, auch wenn an der Kontaktfläche zwischen dem erste Teil und der mindestens einen Anschlageinrichtung eine Normalkraft auf die Kontaktfläche wirkt. Zwar muss die Normalkraft nicht groß sein, denn sie kann z.B. der Gewichtskraft des ersten Teils entsprechen. Doch durch das Lager kann eine leichtgängige drehbare Verbindung bereitgestellt werden, und das Zusammenwirken der einzelnen Komponenten der drehbaren Verbindung kann optimiert werden. Bevorzugt ist die mindestens eine Anschlageinrichtung so zwischen dem ersten Teil und dem zweiten Teil angeordnet, dass der erste Teil nur mit der mindestens einen Anschlageinrichtung in Kontakt ist, sei es direkt oder auch zumindest teilweise mittelbar in Verbindung mit einer Sicherheitseinrichtung, nicht aber mit dem zweiten Teil in Kontakt ist. Der zweite Teil seinerseits ist nur mit der mindestens einen Anschlageinrichtung und dem zweiten Verbindungsbauteil in Kontakt. Mit anderen Worten wirkt der erste Teil (bevorzugt nur) mittels der mindestens einen Anschlageinrichtung mit dem zweiten Teil zusammen.

Wahlweise kann auch eine Gleitfläche durch einen Sicherungsring bereitgestellt werden, insbesondere einen am zweiten Teil oder zweiten Verbindungsbauteil in einer vordefinierten Axialposition gelagerten Sicherungsring, oder einen in einer Nut am ersten Verbindungsbauteil gelagerten Sicherungsring.

Für den Fall dass eine Sicherungseinrichtung vorgesehen ist, kann das (Dreh-)Lager auch zwischen der Sicherungseinrichtung und der Anschlageinrichtung und/oder zwischen dem ersten Teil und der Sicherungseinrichtung gebildet sein, je nachdem, ob eine Relativbewegung zwischen der Sicherungseinrichtung und dem ersten Teil stattfinden soll. Bevorzugt ist die Sicherungseinrichtung in einer vorbestimmten Position am ersten Teil kraft- und/oder formschlüssig fixiert (insbesondere in radialer Richtung auf den ersten Teil gesteckt) und greift axial zwischen den ersten Teil und die (jeweilige) Anschlageinrichtung ein, so dass die Relativbewegung zwischen der Sicherungseinrichtung und der Anschlageinrichtung stattfindet, nicht jedoch zwischen der Sicherungseinrichtung und dem ersten Teil.

Bevorzugt weist der erste Teil an der Unterseite eine Anlagefläche auf, an welcher der erste Teil relativ zur Anschlageinrichtung gleitend verdrehbar ist. Diese Anlagefläche bzw. Gleitfläche ist bevorzugt ringförmig umlaufend ausgebildet. Gemäß einer Variante kann eine solche Anlagefläche oder Gleitfläche auch durch eine/die Sicherungseinrichtung bereitgestellt werden.

Gemäß einem Ausführungsbeispiel weist der erste Teil eine an einer Stirnseite, insbesondere an einer zur (jeweiligen) mindestens einen Anschlageinrichtung hin weisenden Stirnseite, angeordnete Gleitfläche auf und ist eingerichtet, mit der Gleitfläche auf der mindestens einen Anschlageinrichtung gleitend zu drehen. Ferner kann die (jeweilige) Anschlageinrichtung eine an einer Stirnseite, insbesondere an einer zum ersten Teil hin weisenden bzw. vom zweiten Verbindungsbauteil weg weisenden Stirnseite, angeordnete Gleitfläche aufweisen und eingerichtet sein, den ersten Teil mittels der Gleitfläche für eine gleitende Drehbewegung um die Drehachse zu lagern. Die Gleitfläche des ersten Teils und/oder die Gleitfläche der mindestens einen Anschlageinrichtung können dabei bevorzugt z.B. vollständig umlaufend als Kreisringfläche ausgebildet sein, oder auch nur abschnittsweise. Hierdurch kann das Lager für den zweiten Teil auf einfache und kostengünstige Weise bereitgestellt werden. Durch das flächige Aufliegen auf der mindestens einen Anschlageinrichtung kann ein robuster Anschlagmechanismus bereitgestellt werden, welcher sich auf einfache Weise manuell betätigen lässt. Es müssen keine Schrauben oder sonstigen Sicherungsmittel oder Befestigungselemente gelöst werden, allenfalls ein optional vorgesehener Sicherungsring zum axialen Sichern des ersten Teils. Das Ineinandergreifen der Komponenten, also des ersten Teils, der Anschlageinrichtung(en) und des zweiten Teils kann allein aufgrund der Gravitationskraft sichergestellt werden. Die flächige Lagerung auf ringförmigen Stirnflächen kann dabei eine exakte Positionierung der Komponenten relativ zueinander sicherstellen und die drehbare Verbindung sehr robust und leichtlaufend ausgestalten.

Als Gleitfläche ist dabei bevorzugt eine Fläche zu verstehen, welche einen geringen Reibungskoeffizienten für Gleitreibung aufweist, sei es aufgrund einer besonders niedrigen Rauhigkeit bzw. einer besonders glatten Oberfläche, sei es aufgrund eines reibungsarmen Materials mit Schmiereigenschaften. Als Material für die Anschlageinrichtung bzw. den Stellring kann z.B. Zinkdruckguss verwendet werden, sei es mit oder ohne Beschichtung.

Bevorzugt ist die Anschlageinrichtung als ein Stellring mit einer stirnseitig in axialer Richtung in Richtung des zweiten Teils hervorstehenden formschlüssigen Kontur ausgebildet. Als Stellring ist dabei bevorzugt ein (abgesehen von irgendwelchen Anschlägen) rotationssymmetrisches Teil zu verstehen, welches in unterschiedlichen Drehwinkelpositionen positioniert werden kann, z.B. jeweils um 15° versetzt, also z.B. in 24 unterschiedlichen Drehwinkelpositionen.

Bevorzugt ist die Anschlageinrichtung ein einstückiges, in axialer Richtung auf den zweiten Teil aufsteckbares Teil, an welchem der mindestens eine Gegenanschlag bevorzugt in radialer Richtung hervorsteht, insbesondere von einer Außen- oder Innenmantelfläche der Anschlageinrichtung. Hierdurch kann der Platzbedarf in axialer Richtung (die erforderliche Bauhöhe) gering gehalten werden und eine flache Bauform realisiert werden.

Gemäß einem Ausführungsbeispiel weist der anpassbare Anschlagmechanismus mindestens zwei Anschlageinrichtungen auf, welche axial hintereinander (in Reihe) angeordnet sind, wobei jede Anschlageinrichtung einen in axialer Richtung hervorstehenden Gegenanschlag aufweist. Hierdurch kann der Anschlag mit beiden Gegenanschlägen gekuppelt werden. Bevorzugt sind die Gegenanschläge in axialer Richtung überlappend anordenbar. Dies erleichtert das gleichzeitige Kuppeln oder in Eingriff bringen mit mehreren Gegenanschlägen. Bevorzugt sind die Gegenanschläge auf unterschiedlichen Teilkreisen angeordnet. Bevorzugt sind die Gegenanschläge in radialer Richtung gesehen zumindest annähernd deckungsgleich ausgebildet. Dies ermöglicht einen maximal großen Drehwinkel, also einen Drehwinkel, welcher nicht dadurch beschränkt wird, dass bei mehreren Gegenanschlägen jeder Gegenanschlag einen bestimmten Kreisbogen jeweils für sich beansprucht. Der Anschlagmechanismus kann dann selbst bei einer Mehrzahl von Anschlageinrichtungen, z.B. auch drei oder mehr Anschlageinrichtungen, auch so eingestellt werden, als würde nur eine einzige Anschlageinrichtung verwendet. Dies erhöht auch die Flexibilität und Varianz des Anschlagmechanismus.

Bevorzugt sind die beiden Gegenanschläge in axialer Richtung derart korrespondierend zueinander dimensioniert, dass ein jeweiliges freies Ende (insbesondere eine obere Kante) der beiden Gegenanschläge an derselben axialen Position anordenbar ist. Mit anderen Worten: axial überlappend angeordnete Anschlageinrichtungen bzw. Gegenanschläge weisen Gegenanschläge auf, deren jeweiliges freies Ende oder deren Stirnseite in axialer Richtung gesehen an derselben Axialposition anordenbar ist. Hierdurch kann der erste Teil auf konstruktiv vorteilhafte Weise als doppelwandiger Ring bereitgestellt werden, welcher die mindestens zwei Gegenanschläge in axialer Richtung umgreift, also welcher über beide Gegenanschläge gestülpt werden kann, insbesondere auch bei in axialer Richtung schlanker Bauform.

Bevorzugt weist mindestens eine der beiden Anschlageinrichtungen einen in axialer Richtung hervorstehenden Rand auf, mittels welchem die Anschlageinrichtungen relativ zueinander positioniert, insbesondere zentriert werden können. Dies erleichtert das manuelle Einstellen und kann ein gegenseitiges Sichern der Komponenten bewirken.

Bevorzugt weist mindestens eine der beiden Anschlageinrichtungen eine Innenmantelfläche auf, welche geometrisch korrespondierend zu einer entsprechenden Fläche des ersten Verbindungsbauteils ausgebildet ist. Hierdurch kann die (jeweilige) Anschlageinrichtung konzentrisch zum ersten Verbindungsbauteil positioniert werden, insbesondere zentriert werden.

Gemäß einem Ausführungsbeispiel weist eine der Anschlageinrichtungen auf einer Unterseite eine formschlüssige Kontur auf, wobei die andere der Anschlageinrichtungen auf einer Oberseite und einer Unterseite jeweils eine formschlüssige Kontur aufweist. Hierdurch kann die andere der Anschlageinrichtungen als eine zwischengelagerte Anschlageinrichtung zwischen der einen Anschlageinrichtung und dem zweiten Teil axial gelagert werden. Die beidseitige formschlüssige Kontur kann das Einstellen einer bestimmten Drehwinkelposition sowohl in Bezug auf die eine Anschlageinrichtung als auch in Bezug auf den zweiten Teil ermöglichen. Die formschlüssigen Konturen ermöglichen ein geometrisches Zusammenwirken der beiden Anschlageinrichtungen. Bevorzugt sind beide formschlüssigen Konturen vollständig umlaufend vorgesehen, so dass auf flexible Weise eine Vielzahl unterschiedlicher Drehwinkelpositionen eingestellt werden können, wobei eine Kraftübertragung unabhängig von einer bestimmten Drehwinkelposition ist.

Bevorzugt sind die beiden Anschlageinrichtungen in axialer Richtung an geometrisch zueinander korrespondierend ausgebildeten formschlüssigen Konturen verdrehsicher ineinander steckbar. Die formschlüssigen Konturen sind bevorzugt jeweils derart ausgebildet, dass die beiden Anschlageinrichtungen in einer Vielzahl unterschiedlicher Drehwinkelpositionen relativ zueinander verdrehsicher aneinander gelagert werden können.

Gemäß einem Ausführungsbeispiel sind die formschlüssigen Konturen jeweils als eine Vielzahl symmetrisch umlaufend angeordneter, radial ausgerichteter Zähne ausgebildet. Dies ermöglicht eine Vielzahl unterschiedlicher Drehwinkelpositionen, insbesondere in gleichbleibenden Abständen von z.B. 7.5° zueinander.

Gemäß einem Ausführungsbeispiel ist sowohl die formschlüssige Kontur der einen Anschlageinrichtung als auch eine der formschlüssigen Konturen der anderen Anschlageinrichtung geometrisch korrespondierend zu einer/der formschlüssigen Kontur des zweiten Teils ausgebildet. Dies ermöglicht ohne weitere Änderungen oder Zusatzkomponenten, den Anschlagmechanismus wahlweise mit nur einer oder mit zwei (oder noch mehr) Anschlageinrichtungen auszugestalten, insbesondere in Abhängigkeit davon, ob nur der Drehbereich oder auch ein zulässiger Drehwinkel eingestellt werden soll.

Bevorzugt weisen die beiden Anschlageinrichtungen in radialer Richtung zueinander versetzte Gegenanschläge auf, so dass die Gegenanschläge jeweils auf unterschiedlichen Teilkreisen verdrehbar unabhängig voneinander in den unterschiedlichen Drehwinkelpositionen anordenbar sind.

Gemäß einem Ausführungsbeispiel weist der anpassbare Anschlagmechanismus eine Sicherungseinrichtung auf, welche am ersten Teil anordenbar ist und eingerichtet ist, mit der mindestens einen Anschlageinrichtung zusammenzuwirken. Hierdurch kann auf einfache Weise, insbesondere manuell und werkzeuglos, eine Sicherung, insbesondere Axialsicherung, vorgesehen werden. Der erste Teil kann mittels der Sicherungseinrichtung durch axiale Sicherung am ersten Verbindungsbauteil gesichert sein. Indem die mindestens eine Anschlageinrichtung mittels der Sicherungseinrichtung axial relativ zum ersten Teil positioniert und gesichert wird, kann auch die Axialposition der mindestens einen Anschlageinrichtung relativ zum zweiten Verbindungsbauteil gesichert werden.

Bevorzugt ist die Sicherungseinrichtung als halbschalenförmige Kappe ausgebildet, welche auf den ersten Teil aufklippbar ist, insbesondere in radialer Richtung. Eine Halbschale kann auf besonders leichte Weise gehandhabt werden.

Gemäß einem Ausführungsbeispiel ist die Sicherungseinrichtung eingerichtet, eine axiale Verlagerung der mindestens einen Anschlageinrichtung relativ zum ersten Teil zu verhindern. Hierdurch kann eine Axialsicherung auf einfache Weise optional vorgesehen werden, insbesondere manuell. Eine der Anschlageinrichtungen kann mittels der Sicherungseinrichtung gesichert werden, insbesondere mittelbar über den ersten Teil, und weitere Anschlageinrichtungen können über die axial gesicherte Anschlageinrichtung gesichert werden. Der erste Teil kann axial am ersten Verbindungsbauteil gesichert sein und eine relative axiale Verlagerung zwischen den einzelnen Komponenten, also zwischen dem ersten Teil, den Anschlageinrichtungen, dem zweiten Teil sowie zwischen dem zweiten Teil und dem zweiten Verbindungsbauteil verhindern. Die Sicherungseinrichtung kann die manuelle Bedienbarkeit des Anschlagmechanismus auf einfache Weise sicherstellen, insbesondere in Verbindung mit einer oder zwei weiteren Komponenten, nämlich einem im ersten Verbindungsbauteil axial gesicherten Sicherungsring und wahlweise auch einem zwischen dem Sicherungsring und dem ersten Teil angeordneten (Distanz-)Ring, mittels welchem z.B. auch ein axiales Spiel einstellbar ist.

Bevorzugt ist die Sicherungseinrichtung als Halbschale mit einem U-förmigen Querschnittsprofil ausgebildet und weist einen Innenrand auf, welcher zwischen dem ersten Teil und der mindestens einen Anschlageinrichtung anordenbar ist. Bevorzugt umgreift die Halbschale eine Außenmantelfläche des ersten Teils und ist selbst am ersten Teil gesichert, insbesondere in einer vordefinierten Relativposition zum ersten Teil. Bevorzugt ist der Innenrand axial zwischen einer Oberseite der mindestens einen Anschlageinrichtung und einer Unterseite des ersten Teils anordenbar. Um den Halt der Sicherungseinrichtung am ersten Teil zu verbessern, kann der Innenrand beidseitig als Schenkel des U-Profils ausgebildet sein, so dass die Sicherungseinrichtung den ersten Teil schalenartig umgreifen kann. Wahlweise ist der Innenrand nur an einer Unterseite der Sicherungseinrichtung vorgesehen und ausschließlich zwischen dem ersten Teil und der Anschlageinrichtung anordenbar. An dieser Position kann die Axialsicherung der Anschlageinrichtung bevorzugt sichergestellt werden.

Gemäß einem Ausführungsbeispiel weist der anpassbare Anschlagmechanismus ein Dämpfungselement, insbesondere aus Elastomermaterial, auf, welches mit dem zweiten Teil und/oder der mindestens einen Anschlageinrichtung, insbesondere einer stirnseitig in axialer Richtung hervorstehenden formschlüssigen Kontur der jeweiligen Anschlageinrichtung, korrespondiert, welches insbesondere geometrisch korrespondierend zur formschlüssigen Kontur ausgebildet ist. Hierdurch kann sichergestellt werden, dass beim Aufeinandertreffen der Anschläge ein Stoß abgedämpft wird, wodurch die Lebensdauer der drehbaren Verbindung erhöht werden kann und/oder die Stativvorrichtung, insbesondere eine medizintechnische Einrichtung, geschont werden kann. Das Dämpfungselement kann ein Zurückschwingen oder Zurückfedern des Trägers bei einem schlagartigen Anschlagen der Anschläge aneinander verhindern. Als Dämpfungselement ist dabei bevorzugt ein Gummielement mit einer an die jeweilige formschlüssige Kontur angepassten Geometrie zu verstehen. Das Dämpfungselement kann z.B. die Form eines Mäanders aufweisen. Das Dämpfungselement kann als Einlage oder Mantel am zweiten Teil oder an der mindestens einen Anschlageinrichtung angeordnet sein, insbesondere an einer jeweiligen formschlüssigen Kontur.

Gemäß einem Ausführungsbeispiel weist die drehbare Verbindung ein Zwischenelement auf, welches in axialer Richtung gesehen zwischen dem ersten Teil, insbesondere zwischen der Anschlageinrichtung, und dem zweiten Verbindungsbauteil angeordnet ist und mindestens eine formschlüssige Kontur zur drehfesten Verbindung mit der mindestens einen Anschlageinrichtung oder dem zweiten Verbindungsbauteil aufweist, wobei bevorzugt an zwei gegenüberliegenden Stirnflächen des Zwischenelements jeweils eine formschlüssige Kontur angeordnet ist. Hierdurch kann der an den zwei Verbindungsbauteilen angeordnete Anschlagmechanismus noch flexibler ausgelegt werden, und kann insbesondere auch bei gegossenen Buchsen auf einfache Weise vorgesehen werden. Die formschlüssige Kontur kann eine Verdrehsicherung weiterer Elemente, z.B. eines Schleifringinnenteils, sicherstellen. Das Zwischenelement kann auch in Hinblick auf fertigungstechnische Besonderheiten Vorteile liefern. Insbesondere kann die formschlüssige Kontur am zweiten Teil auf einfachere Weise eingebracht werden, speziell an einem Ringabschnitt einer gabelförmigen Buchse. Es kann z.B. auf ein kostenaufwändiges Druckguss-Werkzeug verzichtet werden. Das Zwischenelement kann sicherstellen, dass eine Entformungsschräge an der Buchse derart ausgeglichen wird, dass eine rechtwinklig zur Drehachse ausgerichtete Auflagefläche bereitgestellt werden kann.

Als Zwischenelement ist dabei bevorzugt ein Element zu verstehen, welches sich formschlüssig sowohl mit der mindestens einen Anschlageinrichtung als auch mit dem zweiten Teil verdrehsicher kuppeln lässt. Das Zwischenelement ist ein optional vorgesehenes, zusätzliches Teil, an welchem eine formschlüssige Kontur auf besonders einfache Weise vorgesehen werden kann, bevorzugt an einer Stirnseite. Das Zwischenelement kann z.B. auch aus fertigungstechnischen Gründen vorgesehen werden. Eine mechanische Bearbeitung des Zwischenelements, insbesondere das Einbringen der formschlüssigen Kontur(en), kann auf einfache Weise erfolgen. Das Zwischenelement lässt sich auf einfache Weise handhaben und weist gut zugängliche Oberflächen auf. Bevorzugt ist die (jeweilige) formschlüssige Kontur durch Nuten gebildet, welche sich in radialer Richtung erstrecken. Die Nuten können sich entlang des gesamten Zwischenelements erstrecken. Wahlweise können die Nutzen abschnittsweise (als kurze Nuten) in Kombination mit Federn vorgesehen sein.

Gemäß einem Ausführungsbeispiel ist das Zwischenelement als Scheibe, insbesondere ringförmige Scheibe ausgebildet. Hierdurch kann das Zwischenelement in Reihe mit den anderen Komponenten um das erste Verbindungsbauteil herum angeordnet werden. Der flache Aufbau als Scheibe kann dabei auch einen geringen Platzbedarf in axialer Richtung sicherstellen.

Gemäß einem Ausführungsbeispiel ist das Zwischenelement keilförmig mit einer in Bezug auf die axiale Richtung uneinheitlichen axialen Abmessung bzw. Dicke ausgebildet. Hierdurch kann die drehbare Verbindung auf einfache Weise in Verbindung mit einer gegossenen Buchse verwendet werden, an welcher eine Entformungsschräge vorgesehen ist. Mittels der Keilform kann die Entformungsschräge ausgeglichen werden, so dass die beiden Teile bzw. die mindestens eine Anschlageinrichtung axial ausgerichtet aneinander angeordnet werden können. Mit anderen Worten: die keilförmige Geometrie ist eingerichtet, eine Entformungsschräge der Buchse auszugleichen.

Die zuvor genannte Aufgabe wird auch durch ein Tragsystem für eine Stativvorrichtung zur Anordnung in einem Operationssaal und zum Positionieren einer medizintechnischen Einrichtung im Operationssaal gelöst, welches eine erfindungsgemäße drehbare Verbindung sowie das erste Verbindungsbauteil, insbesondere in Form einer Spindel, und das zweite Verbindungsbauteil, insbesondere in Form einer Buchse, aufweist.

Als Tragsystem sind dabei bevorzugt diejenigen Komponenten der Stativvorrichtung zu verstehen, welche zumindest teilweise auch eine Funktion zum Halten und Positionieren der medizintechnischen Einrichtung übernehmen. Das Tragsystem kann eine Vielzahl von jeweils relativ zueinander verlagerbaren bevorzugt starren Armen bzw. Trägern sowie eine Vielzahl von Hebeln, Gelenken oder Lagern umfassen.

Als medizintechnische Einrichtung ist dabei bevorzugt eine Leuchte, ein Monitor und/oder eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Gemäß einem Ausführungsbeispiel ist das zweite Verbindungsbauteil als gabelförmige Buchse ausgebildet, wobei die mindestens eine Anschlageinrichtung und der erste Teil und der zweite Teil zwischen zwei Ringabschnitten der Buchse an einem der beiden Ringabschnitte angeordnet sind, und wobei der zweite Teil einen Formschlussabschnitt aufweist, insbesondere eine Gabel, mittels welchem der zweite Teil verdrehsicher am zweiten Verbindungsbauteil, insbesondere an einem Steg, positionierbar ist. Der Formschlussabschnitt kann sich seitlich radial nach außen vom zweiten Teil erstrecken. Bei dieser Anordnung kann der zweite Teil auf einfache Weise an der Buchse verdrehsicher gelagert werden. Die Buchse selbst muss dabei nicht bearbeitet werden, muss insbesondere nicht spanend bearbeitet werden, oder muss auch nicht durch Fräsen bearbeitet werden. Bei dieser Anordnung bleiben zudem alle Komponenten gut zugänglich, insbesondere da der zweite Teil auf dem Ringabschnitt der Buchse aufliegt. Diese Anordnung liefert auch die Möglichkeit, den anpassbaren Anschlagmechanismus auf einfache Weise nachzurüsten, ohne dafür bei einer Buchse eine formschlüssige Kontur einarbeiten zu müssen, insbesondere durch nachträgliche spanende Bearbeitung wie z.B. Fräsen.

Gemäß einem Ausführungsbeispiel ist das zweite Verbindungsbauteil als Buchse, insbesondere gabelförmige Buchse, ausgebildet, wobei zumindest die mindestens eine Anschlageinrichtung und der zweite Teil und bevorzugt auch der erste Teil in der Buchse angeordnet sind, insbesondere zwischen zwei Ringabschnitten der Buchse, bevorzugt in einem der beiden Ringabschnitte. Die drehbare Verbindung kann ferner ein Zwischenelement aufweisen, welches in die Buchse, insbesondere in einen der beiden Ringabschnitte, eingelegt ist. Hierdurch kann eine drehbare Verbindung bereitgestellt werden, deren mindestens einen Anschlageinrichtung gut zugänglich ist, was das Einstellen des Drehwinkels bzw. Drehwinkelbereichs erleichtert. Die einzelnen Komponenten können auf einfache Weise in die Buchse eingelegt werden, insbesondere von der Seite in radialer Richtung. Dabei kann auch ein zusätzliches Zwischenelement in die Buchse, insbesondere in einen der beiden Ringabschnitte, eingelegt werden, insbesondere um eine Entformungsschräge auszugleichen und/oder eine einfache oder kostengünstige Herstellung der formschlüssigen Konturen zu ermöglichen. Die einzelnen Komponenten können auch auf einfache Weise in axialer Richtung relativ zueinander verlagert werden, um den Drehwinkel bzw. Drehbereich einzustellen.

Durch das Zwischenelement kann auch eine in axialer Richtung besonders flache Bauform der drehbaren Verbindung sichergestellt werden, was z.B. bei Zentralachsen vorteilhaft ist, welche in axialer Richtung meist bereits eine beträchtliche Ausdehnung aufweisen.

Die zuvor genannte Aufgabe wird auch durch eine Stativvorrichtung zur Anordnung in einem Operationssaal und zum Positionieren einer medizintechnischen Einrichtung im Operationssaal gelöst, welche eine erfindungsgemäße drehbare Verbindung oder das zuvor beschriebene Tragsystem mit der erfindungsgemäßen drehbaren Verbindung umfasst.

In einer speziellen Ausgestaltungsform umfasst die Stativvorrichtung zur Anordnung in einem Operationssaal und zum Positionieren einer medizintechnischen Einrichtung im Operationssaal ein Tragsystem mit wenigstens einem Träger, insbesondere Tragarm, mit einer Buchse, die relativ zu einem ortsfest angeordneten Teil der Stativvorrichtung oder zu einem weiteren Träger der Stativvorrichtung verdrehbar um eine Drehachse an einer Spindel an einer drehbaren Verbindung, insbesondere einer erfindungsgemäßen drehbaren Verbindung, gelagert ist, wobei die drehbare Verbindung einen anpassbaren Anschlagmechanismus aufweist, der zwischen der Spindel und der relativ zur Spindel verdrehbar um die Drehachse gelagerten Buchse angeordnet ist und eingerichtet ist, mindestens zwei unterschiedliche relative Drehwinkel der Buchse relativ zur Spindel oder mindestens zwei unterschiedliche Drehbereiche festzulegen, wobei der anpassbare Anschlagmechanismus aufweist:
- einen ersten Teil in Form eines doppelwandigen Anschlagrings, welcher verdrehsicher an der Spindel gelagert ist und einen Anschlag in Form eines Stegs aufweist;
- einen zweiten Teil, welcher verdrehsicher mit der Buchse verbunden ist;
wobei der Anschlagring relativ zum zweiten Teil verdrehbar gelagert ist und axial relativ zur Spindel verlagerbar angeordnet ist;
wobei der anpassbare Anschlagmechanismus zwei in Richtung der Drehachse axial verlagerbar angeordnete Stellringe mit jeweils einem Gegenanschlag aufweist, welche in axialer Richtung gesehen zwischen den beiden Teilen angeordnet sind und mit den beiden Teilen zusammenwirken, wobei der jeweilige Gegenanschlag mit dem Steg zusammenwirkt und wobei die Stellringe eingerichtet sind, mittels des Gegenanschlags jeweils die unterschiedlichen relativen Drehwinkel oder Drehbereiche festzulegen, und wobei der Anschlagring die Stellringe in axialer Richtung überlappt und den jeweiligen Gegenanschlag radial innen und außen umgreift, insbesondere indem die Gegenanschläge in einer Ringkavität des Anschlagrings aufgenommen werden. Mittels eines solchen Anschlagmechanismus kann die Stativvorrichtung, insbesondere einzelne Träger relativ zueinander, in einem auf flexible Weise einstellbaren Aktionsradius positioniert werden. Der oder die Gegenanschläge können am zweiten Teil versetzt (verdreht) werden, sei es relativ zueinander und/oder relativ zum zweiten Teil, um zusammen eine einzige geeignete Drehwinkelposition oder jeweils einzeln unterschiedliche Drehwinkelpositionen festzulegen, insbesondere hinsichtlich einer spezifischen Anordnung der Stativvorrichtung relativ zu weiteren Komponenten im Operationssaal.

Als Träger ist dabei bevorzugt ein Ausleger oder Tragarm zu verstehen, welcher sich in einer bestimmten Richtung erstreckt und den gewünschten Aktionsradius für die unterschiedlichen Soll-Positionen der medizintechnischen Einrichtung sicherstellen kann, insbesondere durch eine Drehbewegung um eine drehbare Verbindung. Der Träger kann wahlweise auch in der Höhe verschwenkt werden und/oder in der Höhe translatorisch verlagert werden. Der Träger kann auch eine teleskopische Vorrichtung mit einem (zusätzlichen) Bewegungsfreiheitsgrad in translatorischer Richtung entlang der Längsachse des Trägers sein. Der Träger kann zumindest teilweise z.B. durch ein Stranggussprofil, insbesondere ein Aluminium-Stranggussprofil gebildet sein.

Mittels der mindestens einen Anschlageinrichtung ist ein Drehbereich oder ein Betrag eines Drehwinkels der drehbaren Verbindung, insbesondere ein zulässiger relativer Drehwinkel der beiden Verbindungsbauteile zueinander definierbar.

Bevorzugt ist der zweite Teil an dem bzw. an einem der Träger im Bereich der drehbaren Verbindung angeordnet. An einem der Träger kann ortsfest eine Kontur oder ein Anschlag fixiert sein, womit der Träger in Bezug auf den anderen Träger oder in Bezug auf irgendeinen anderen ortsfest angeordneten Teil in den unterschiedlichen Drehwinkelpositionen positionierbar ist.

Die zuvor genannte Aufgabe wird auch gelöst durch ein Verfahren zum Einstellen eines anpassbaren Anschlagmechanismus einer drehbaren Verbindung zwischen zwei Verbindungsbauteilen für eine Stativvorrichtung zur Anordnung in einem Operationssaal, wobei der Anschlagmechanismus einen ersten Teil, einen zweiten Teil und mindestens eine Anschlageinrichtung mit jeweils einem Gegenanschlag aufweist, wobei der zweite Teil verdrehsicher an einem/dem verdrehbar um eine Drehachse gelagerten zweiten Verbindungsbauteil gelagert ist, mit den Schritten:
- Lösen eines formschlüssigen Eingriffs zwischen der mindestens einen Anschlageinrichtung und dem zweiten Teil durch axiales Verlagern des ersten Teils und der mindestens einen Anschlageinrichtung entlang der Drehachse und entlang des ersten Verbindungsbauteils; und
- Festlegen eines Drehbereichs oder eines relativen Drehwinkels der Verbindungsbauteile relativ zueinander durch axiales Zurückverlagern des ersten Teils zusammen mit der mindestens einen Anschlageinrichtung und formschlüssiges Eingreifen der mindestens einen Anschlageinrichtung im zweiten Teil in einer geänderten Drehwinkelposition. Hierdurch ergeben sich insbesondere die im Zusammenhang mit der drehbaren Verbindung beschriebenen Vorteile.

Gemäß einer vorteilhaften Ausführungsform betrifft das Verfahren einen Anschlagmechanismus mit mindestens zwei Anschlageinrichtungen und umfasst ferner die Schritte:
- Lösen eines formschlüssigen Eingriffs zwischen dem ersten Teil, insbesondere einem Anschlag des ersten Teils, und einem/dem jeweiligen Gegenanschlag der mindestens zwei Anschlageinrichtungen durch axiales Verlagern des ersten Teils relativ zu den mindestens zwei Anschlageinrichtungen, insbesondere in Richtung der Drehachse;
- Lösen eines formschlüssigen Eingriffs zwischen den mindestens zwei Anschlageinrichtungen durch axiales Verlagern der Anschlageinrichtungen relativ zueinander; und
- Verdrehen der Anschlageinrichtungen, insbesondere der Gegenanschläge, relativ zueinander und dann formschlüssiges Eingreifen der Anschlageinrichtungen durch axiales Zurückverlagern der Anschlageinrichtungen relativ zueinander, insbesondere durch Ineinanderstecken, in einer geänderten Drehwinkelposition, bevor die Anschlageinrichtungen oder zumindest eine der Anschlageinrichtungen wieder in formschlüssigen Eingriff mit dem zweiten Teil gebracht werden. Hierdurch kann auf einfache Weise ein Drehwinkel und auch eine Drehposition eingestellt werden, insbesondere bei hoher Flexibilität und Varianz des Anschlagmechanismus. Ferner ergeben sich dabei auch einzelne im Zusammenhang mit der drehbaren Verbindung beschriebene Vorteile.

In den nachfolgenden Zeichnungsfiguren wird die Erfindung anhand von Ausführungsbeispielen näher erläutert. Es zeigen:
- Figur 1A: in schematischer Darstellung in perspektivischer Ansicht eine drehbare Verbindung gemäß einem Ausführungsbeispiel der Erfindung;
- Figur 1B: in einer Schnittansicht die drehbare Verbindung gemäß dem in der Fig. 1A gezeigten Ausführungsbeispiel;
- Figur 1C: in einer perspektivischen Seitenansicht die drehbare Verbindung gemäß dem in der Fig. 1A gezeigten Ausführungsbeispiel in Explosionsdarstellung;
- Figur 2A: in einer perspektivischen Seitenansicht Komponenten eines anpassbaren Anschlagmechanismus für eine drehbaren Verbindung gemäß dem in der Fig. 1A gezeigten Ausführungsbeispiel;
- Figur 2B: in einer perspektivischen Seitenansicht die Komponenten des in der Fig. 2A gezeigten Anschlagmechanismus in Explosionsdarstellung;
- Figur 3A: in einer perspektivischen Seitenansicht Komponenten eines anpassbaren Anschlagmechanismus für ein weiteres Ausführungsbeispiel einer erfindungsgemäßen drehbaren Verbindung;
- Figur 3B: in einer perspektivischen Seitenansicht die Komponenten des in der Fig. 3A gezeigten Anschlagmechanismus in Explosionsdarstellung;
- Figur 4: in einer perspektivischen Seitenansicht ein Dämpfungselement für eine drehbare Verbindung gemäß einem der Ausführungsbeispiele der Erfindung;
- Figur 5A: in einer perspektivischen Seitenansicht ein Zwischenelement einer drehbaren Verbindung gemäß einem weiteren Ausführungsbeispiel der Erfindung;
- Figur 5B: in einer Seitenansicht das in der Fig. 5A gezeigte Zwischenelement;
- Figur 6: in schematischer Darstellung in perspektivischer Ansicht eine gabelförmige Buchse, welche zur Anordnung einer drehbaren Verbindung gemäß einem der Ausführungsbeispiele der Erfindung eingerichtet ist;
- Figuren 7A, 7B: jeweils in schematischer Darstellung in perspektivischer Ansicht eine drehbare Verbindung gemäß einem weiteren Ausführungsbeispiel der Erfindung in Explosionsdarstellung;
- Figur 7C: in perspektivischer Ansicht die in den Figuren 7A, 7B gezeigte drehbare Verbindung in einem montierten Zustand;
- Figur 7D: in einer Schnittansicht die in den Figuren 7A, 7B, 7C gezeigte drehbare Verbindung; und
- Figuren 8A, 8B: jeweils in schematischer Darstellung in perspektivischer Ansicht eine drehbare Verbindung gemäß einem weiteren Ausführungsbeispiel der Erfindung.

Im Zusammenhang mit der Beschreibung der folgenden Figuren wird bei einzelnen Bezugszeichen, falls sie nicht explizit im Zusammenhang mit einer bestimmten Figur erläutert werden, auf die weiteren Figuren verwiesen.

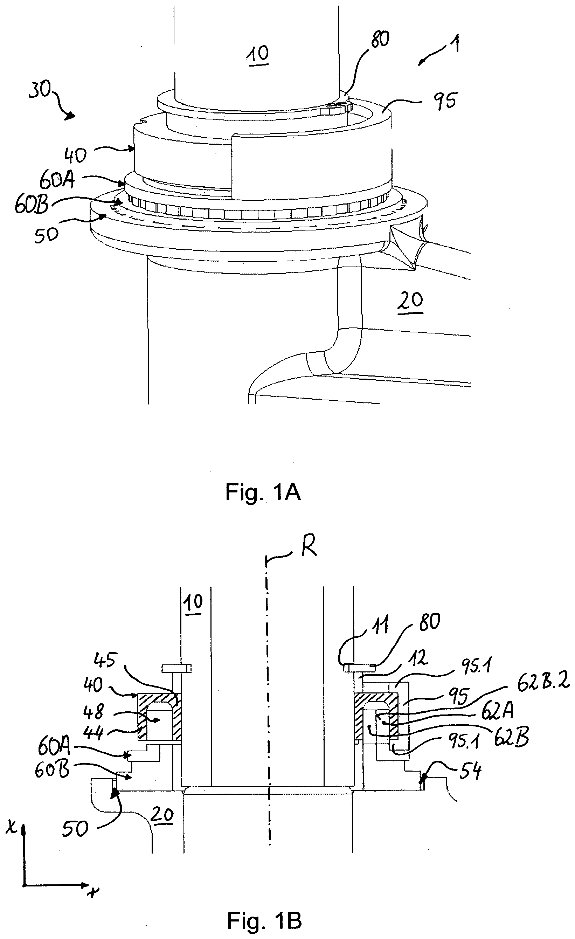

In der Figur 1A ist eine drehbare Verbindung 1 gezeigt, welche zwischen einem ersten Verbindungsbauteil 10, insbesondere einer Spindel, und einem zweiten Verbindungsbauteil 20, insbesondere einer Buchse, angeordnet ist. Die drehbare Verbindung 1 umfasst einen anpassbaren Anschlagmechanismus 30 mit einem ersten Teil (Anschlagring) 40, einer ersten Anschlageinrichtung (Stellring) 60A, einer zweiten Anschlageinrichtung (Stellring) 60A und einem zweiten Teil (Zahnkranz) 50. An der Spindel 10 ist ein Sicherungsring 80 gelagert, welcher eine axiale Verlagerung des Anschlagmechanismus 30 einschränken oder verhindern kann, insbesondere in Verbindung mit einer Sicherungseinrichtung (Halbschale oder Kappe) 95.

Die Spindel 10 ist drehbar um eine Drehachse R angeordnet, wie in Figur 1B gezeigt. In der Spindel 10 ist eine Nut 11 zur Aufnahme des Sicherungsrings 80 vorgesehen. Axial zwischen dem Sicherungsring 80 und einer Oberseite des Anschlagrings 40 wirkt ein Ring 12, insbesondere zur optionalen axialen Sicherung der beiden Stellringe 60A, 60B. Der Anschlagrings 40 weist ein U-förmiges Querschnittsprofil auf und umgreift jeweils einen Gegenanschlag 62A, 62B der Stellringe 60A, 60B in axialer Richtung radial innen und außen mit einer Außenwand 44 und einer Innenwand 45. Zwischen der Außenwand 44 und der Innenwand 45 ist eine Ringkavität 48 gebildet, in welcher die Gegenanschläge 62A, 62B in unterschiedlichen Drehwinkelpositionen anordenbar sind. Die beiden Gegenanschläge 62A, 62B überlappen einander in axialer Richtung. Ein oberes freies Ende bzw. eine Stirnseite der Gegenanschläge 62A, 62B ist an derselben Axialposition angeordnet. In radialer Richtung sind die Gegenanschläge 62A, 62B direkt aneinanderliegend angeordnet. Ein Außenflächenabschnitt 62B.2 des Gegenanschlags 62B des unteren Stellrings 60B liegt innen an einem geometrisch korrespondierenden Innenflächenabschnitt des Gegenanschlags 62A des unteren Stellrings 60A an. Bevorzugt ist der Außenflächenabschnitt 62B.2konvex nach außengewölbt, insbesondere entsprechend eines Radius eines Teilkreises, auf welchem der Außenflächenabschnitt 62B.2 angeordnet ist.

Die Stellringe 60A, 60B sind aufeinanderliegend angeordnet, wobei der untere Stellring 60B auf dem Zahnkranz 50 aufliegt. Der Zahnkranz 50 ist innerhalb der Buchse 20 angeordnet oder ist ein Teil der Buchse, kann also (wie dargestellt) durch die Buchse gebildet sein. Eine formschlüssige Kontur 54 des Zahnkranzes 50 weist in axialer Richtung zum unteren Stellring 60B und ist geometrisch korrespondierend zu einer entsprechenden formschlüssigen Kontur des unteren Stellrings 60B oder auch des oberen Stellrings 60A ausgebildet.

Die Sicherungskappe 95 weist einen beidseitig (oben und unten) nach innen hervorstehenden Innenrand 95.1 auf. Der Innenrand 95.1 greift radial (also in Richtung oder entgegen der Richtung der angedeuteten radialen r-Achse) zwischen dem Anschlagring 40 und dem oberen Stellring 60A ein und sichert den oberen Stellring 60A gegen eine axiale Verlagerung nach oben, also gegen eine Verlagerung in x-Richtung. Die axiale Sicherung kann in Verbindung mit dem Ring 12 sichergestellt werden. Dabei kann der Anschlagring 40 mittels des Innenrandes 95.1 der Sicherungskappe 95 auf dem oberen Stellring 60A gelagert sein. Zusätzlich oder alternativ kann eine Lagerung des Anschlagrings 40 auch auf einem zusätzlichen Absatz oder Gleitelement oder irgendeinem weiteren Sicherungsring erfolgen, welcher z.B. an der Spindel 10 oder auf der Buchse 20 axial gesichert ist. Bevorzugt liegt ein (jeweiliger) Stellring an der Spindel an und wird über die Spindel zentriert.

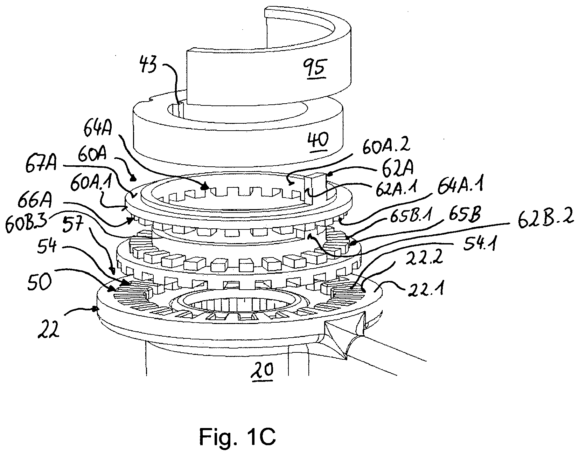

In der Figur 1C ist die integrierte Anordnung des Zahnkranzes 50 in der Buchse 20 erkennbar. Die Buchse 20 weist einen Ringabschnitt 22 auf, an welchem der Zahnkranz 50 ausgebildet ist. Der Ringabschnitt weist eine eben Stirnfläche 22.1 auf, welche eine Oberseite 57 des Zahnkranzes 50 bildet. Der Zahnkranz 50 weist eine Vielzahl von Zähnen 54.1 auf, welche sich in radialer Richtung zwischen einer nach innen weisenden Mantelfläche 22.2 des Ringabschnitts 22 und einer Durchführung für die Spindel 10 erstrecken. An der Mantelfläche 22.2 kann der zweite Stellring 60B zentriert werden.

Der obere Stellring 60A weist an einer Unterseite 66A eine formschlüssige Kontur 64A auf, welche aus einzelnen Zähnen 64A.1 gebildet ist. Die formschlüssige Kontur 64A ist korrespondierend zu einer formschlüssigen Kontur 64B mit entsprechenden Zähnen 64B.1 des unteren Stellrings 60B ausgebildet. Der Gegenanschlag des unteren Stellrings 60B ist radial innen vom Gegenanschlag 62A des oberen Stellrings 60A angeordnet. Der Gegenanschlag 62A des oberen Stellrings 60A (und entsprechend auch der Gegenanschlag des unteren Stellrings 60B) weist eine Seitenfläche (Radialflanke) 62A.1 in Form einer ebenen Anschlagsfläche auf. Der obere Stellring 60A weist eine Innenmantelfläche 60A.2 auf, mittels welcher der obere Stellring 60A am unteren Stellring 60B zentriert werden kann. Mit anderen Worten überlappen nicht nur die Gegenanschläge oder die formschlüssigen Konturen, sondern auch korrespondierende Mantelflächen der Stellringe.

Der obere Stellring 60A weist eine Stirnfläche bzw. Oberseite 67A auf, an welcher ein ringförmiger Flächenabschnitt ausgebildet ist, auf welchem die Sicherungskappe 95 gelagert werden kann. Der ringförmige Flächenabschnitt weist bevorzugt einen geringen Reibungswiderstand auf und kann als Gleitfläche oder Gleitlagerfläche bezeichnet werden, insbesondere um eine widerstandarme relative Drehbewegung der Sicherungskappe 95 zu ermöglichen. Eine Außenmantelfläche 60A.1 des oberen Stellrings 60A ist bevorzugt auf demselben Teilkreis wie eine Außenmantelfläche der Sicherungskappe 95 angeordnet. Dies ermöglicht eine Sichtprüfung dahingehend, ob die Sicherungskappe 95 korrekt positioniert ist.

Aus der Figur 1C geht ferner hervor, dass der anpassbare Anschlagmechanismus 30 an vier axial stirnseitigen Schnittstellen 57, 66A, 66B, 67B jeweils eine formschlüssige Kontur aufweist. Dabei weist der untere Stellring 60B beidseitig eine formschlüssige Kontur auf.

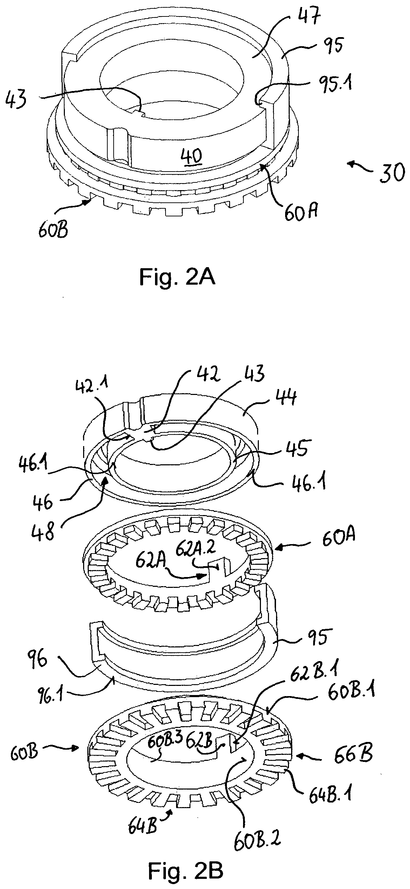

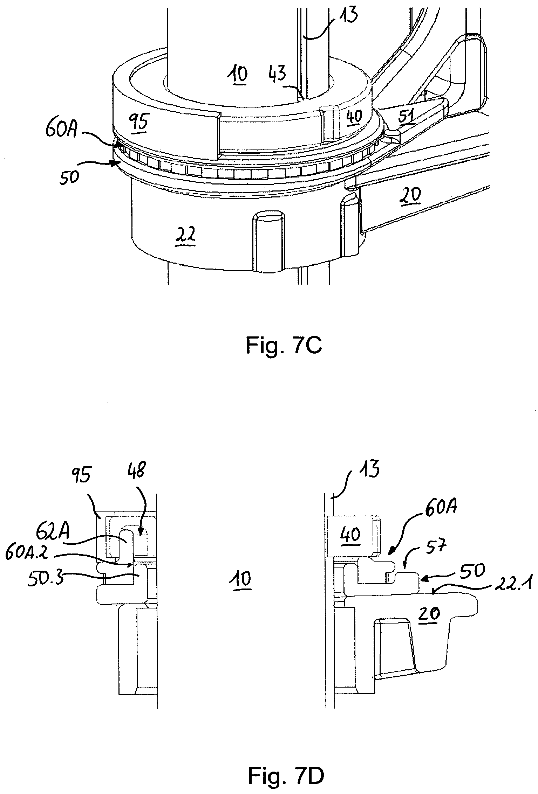

In der Figur 2A ist der Anschlagring 40 in einer perspektivischen Ansicht gezeigt. Der obere Innenrand 95.1 der Sicherungskappe 95 wirkt mit einer Oberseite 47 des Anschlagrings 40 zusammen. An einer Innenmantelfläche des Anschlagrings 40 ist ein Formschlusselement 43, insbesondere eine Feder, angeordnet, welches eingerichtet ist, in eine korrespondierende Nut der Spindel (nicht dargestellt; vgl. Fig. 7c) einzugreifen. Die Feder 43 erstreckt sich in axialer Richtung entlang der gesamten Innenmantelfläche und schließt bündig mit der Oberseite 47 ab.

Die Figur 2B zeigt den Anschlagring 40 und einen/den einzigen Anschlag 42 mit Blick auf eine Unterseite 46. Der Anschlag 42 weist zwei gegenüberliegende Seitenflächen 42.1 auf, die bevorzugt eben ausgebildet sind und sich in radialer Richtung erstrecken. Der Anschlag 42 ist als sich zwischen den Wänden 44, 45 erstreckendes Schott ausgebildet. Der Anschlagring 40 weist nur einen einzigen Anschlag 42 auf. Die Seitenflächen 42.1 können auch als Radialflanken oder Anschlagflächen bezeichnet werden, welche mit den Gegenanschlägen 62A, 62B zusammenwirken können. Der Gegenanschlag 62A des oberen Steilrings 60A weist einen konkav radial nach außen gewölbten Innenflächenabschnitt 62A.2 auf, welcher an einem korrespondierenden Außenflächenabschnitt des Gegenanschlags 62b des unteren Stellrings 60B zur Anlage kommen kann. Diese Anordnung kann z.B. ein gegenseitiges Führen und Stützen der beiden Stellringe aneinander begünstigen und in Verbindung mit der Ringkavität 48 auch eine kompakte Bauform ermöglichen. Der Innenflächenabschnitt 62A.2 des Gegenanschlags 62A des oberen Stellrings 60A erstreckt sich in axialer Richtung auf demselben Teilkreis wie die Innenmantelfläche des oberen Stellrings 60A. Der Innenflächenabschnitt 62A.2 ist ein Flächenabschnitt der Innenmantelfläche. Hierdurch können die Stellringe ineinander angeordnet werden und aneinander zentriert werden. Diese Ausgestaltung erleichtert ein Ineinanderstecken der Stellringe.

An der Unterseite 46 des Anschlagrings 40 sind zwei ringförmige Flächenabschnitte, insbesondere Gleitflächen 46.1 angeordnet, die jeweils einer der Wände 44, 45 zugeordnet sind. Die Gleitfläche der Außenwand 44 korrespondiert mit der Sicherungskappe 95, wobei nicht notwendigerweise eine Relativbewegung zwischen der Sicherungskappe 95 und dem Anschlagring 40 erfolgen muss, und die Gleitfläche der Innenwand 45 kann mit einem optional vorsehbaren Gleitring (vgl. Fig. 1 B) korrespondieren. Die Sicherungskappe 95 weist eine Unterseite 96 auf, an welcher eine Anlagefläche, insbesondere ringförmige Gleitfläche 96.1 ausgebildet ist. Diese Gleitfläche 96.1 kann auf der Oberseite 67A des oberen Stellrings 60A zur Anlage gebracht werden.

Wie auch der Gegenanschlag des oberen Stellrings weist der Gegenanschlag 62B des unteren Stellrings 60B eine Seitenfläche (Radialflanke) 62B.1 auf. Ein Innenflächenabschnitt des Gegenanschlags 62B des unteren Stellrings 60B erstreckt sich in axialer Richtung auf demselben Teilkreis wie eine Innenmantelfläche 60B.2 des unteren Stellrings 60B. Der Gegenanschlag 62B des unteren Stellrings 60B erstreckt sich in axialer Richtung von einem in axialer Richtung hervorstehenden Rand 60B.3. Durch diese Ausgestaltung kann ein Zusammenstecken der Stellringe 60A, 60B auf einfache Weise erfolgen. Der obere Stellring 60A kann am Gegenanschlag 62B entlang geschoben werden und über den Rand 60B.3 geschoben werden. Der untere Stellring 60B weist an einer Unterseite 66B eine erste formschlüssige Kontur 64B auf, welche durch Zähne 64B.1 gebildet ist, die zwischen einer Außenmantelfläche 60B.1 und der Unterseite 66B in radialer Richtung in den Stellring 60B eingebracht sind.

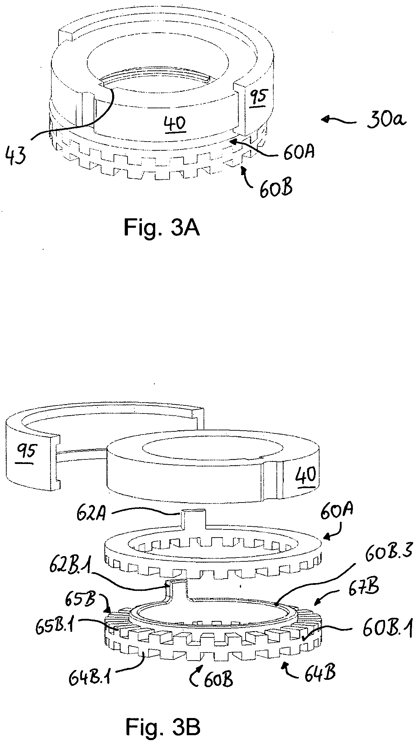

In den Figuren 3A und 3B ist ein weiteres Ausführungsbeispiel eines anpassbaren Anschlagmechanismus 30a gezeigt. Die beiden Stellringe 60A, 60B weisen jeweils eine Außenmantelfläche auf, die auf demselben Teilkreis angeordnet sind. In der Figur 3B ist eine zweite formschlüssige Kontur 65b mit einzelnen Zähnen 65.1 gezeigt, die an einer Oberseite bzw. an einem Stirnflächenabschnitt des unteren Stellrings 60B angeordnet ist. Auch die in den weiteren Figuren gezeigten unteren Stellringe weisen eine solche zweite formschlüssige Kontur auf, oder auch eine davon geometrisch abgewandelte formschlüssige Kontur. Durch dieses Ausführungsbeispiel kann eine einfache formschlüssige Verbindung bereitgestellt werden, durch die sich eine feine Drehwinkeleinstellung realisieren lässt. Zudem lässt sich dieses Ausführungsbeispiel auf einfache Weise in Verbindung mit einem Gussteil realisieren.

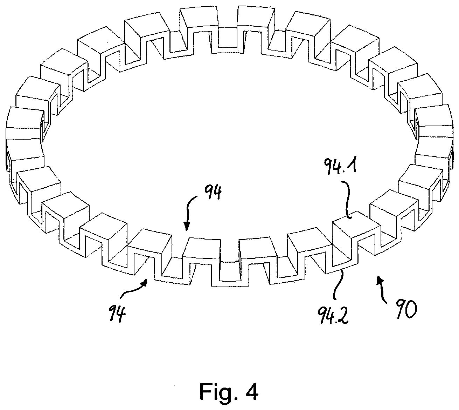

In der Figur 4 ist ein Dämpfungselement 90 gezeigt. Das Dämpfungselement 90 ist ein Gummielement mit einer an die jeweilige formschlüssige Kontur der Stellringe angepassten Geometrie. Das Dämpfungselement weist die Form eines Mäanders auf. Eine formschlüssige Kontur 94 am Dämpfungselement 90 ist an beiden Stirnseiten des Dämpfungselements 90 ausgebildet. Die formschlüssige Kontur 94 weist in beiden axialen Richtungen eine Verzahnungs-Geometrie mit Zähnen 94.1 und 94.2 auf. Die formschlüssige Kontur 94 ist korrespondierend sowohl zu der formschlüssigen Kontur des oberen Stellrings als auch zu der zweiten formschlüssigen Kontur des unteren Stellrings 60 ausgebildet.

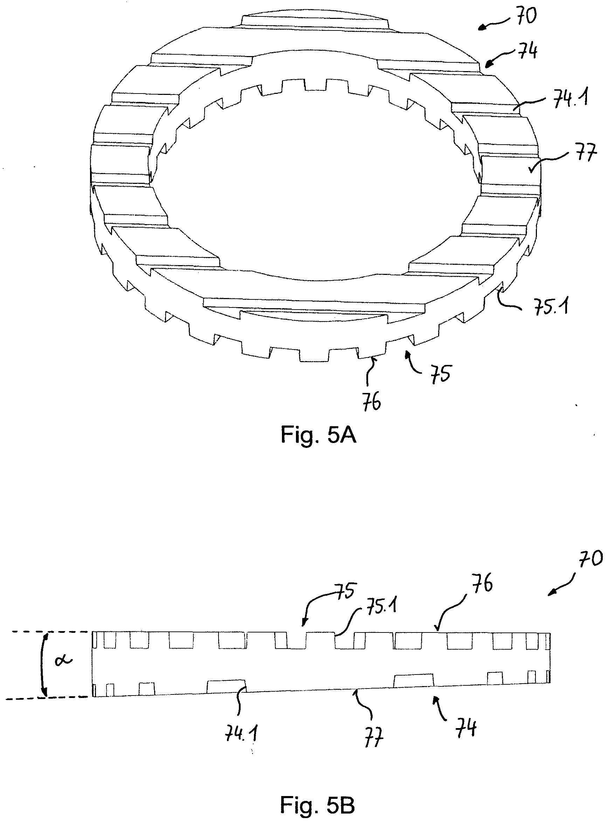

In der Figur 5A ist ein Zwischenelement 70 gezeigt, welches an einer Stirnseite (wie dargestellt an der Oberseite) eine erste formschlüssige Kontur 74 und an einer anderen Stirnseite (wie dargestellt an der Unterseite) eine zweite formschlüssige Kontur 75 aufweist. Die formschlüssigen Konturen 74, 75 weisen jeweils einzelne Nuten 74.1, 75.1 auf. Die an einer ersten Stirnfläche 76 angeordneten Nuten 75.1 erstrecken sich in radialer Richtung. Die Nuten 75.1 sind bevorzugt in einem einheitlichen Winkel zueinander angeordnet, also in Umfangsrichtung gesehen in einheitlichem Abstand zueinander. Die an einer zweiten Stirnfläche 77 angeordneten Nuten 74.1 erstrecken sich linear geradlinig und sind bevorzugt parallel zueinander ausgerichtet. Die Nuten 74.1 weisen bevorzugt einen einheitlichen Abstand zueinander auf. Das Zwischenelement 70 ist ringförmig ausgebildet, und die Stirnflächen 76, 77 sind eben bzw. plan ausgebildet. Das Zwischenelement 70 kann als ringförmige Scheibe beschrieben werden.

In der Figur 5B ist das Zwischenelement 70 in einer Seitenansicht gezeigt. Aus der Figur 5B geht hervor, dass die erste Stirnfläche 76 in einem Winkel α zur zweiten Stirnfläche 77 angeordnet ist, entsprechend der Neigung einer Entformungsschräge. Der Winkel α liegt bevorzugt im Bereich von 1,5°. Die Stirnflächen 76, 77 sind nicht parallel. Vielmehr ist das Zwischenelement 70 keilförmig ausgebildet, insbesondere als keilförmige Ringscheibe. Hierdurch kann eine in der Buchse gebildete Entformungsschräge ausgeglichen werden. Dabei entspricht die Stirnfläche 76 bevorzugt derjenigen Stirnfläche, welche mit dem unteren Stellring in Eingriff gebracht wird.

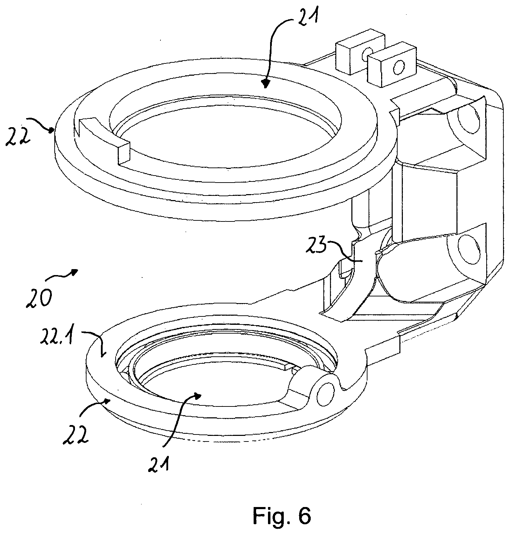

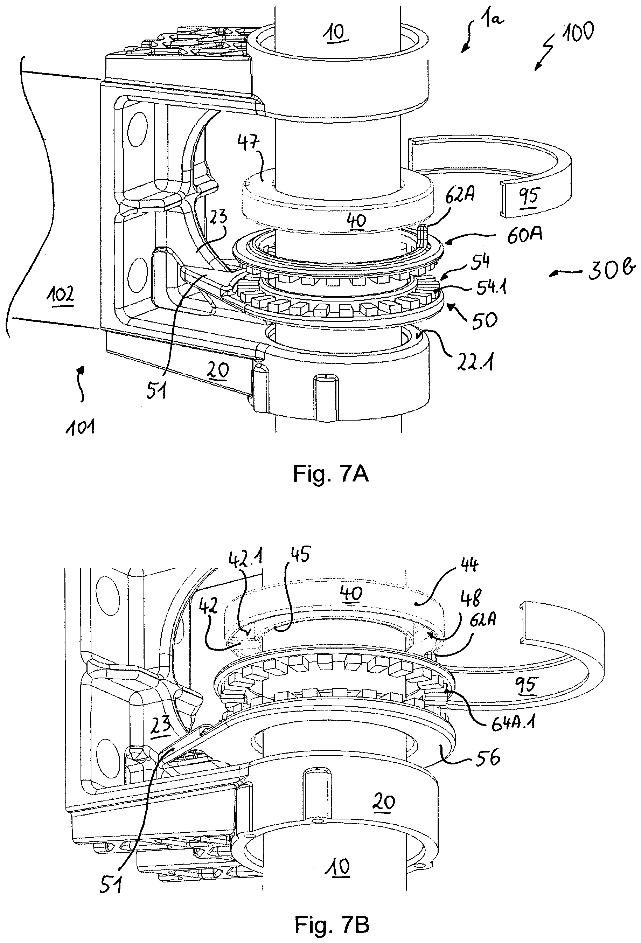

In der Figur 6 eine Ausführungsform einer gabelförmigen Buchse 20 gezeigt. Die Buchse 20 weist zwei Ringabschnitte 22 auf, an welchen jeweils eine Durchführung 21 für eine Spindel ausgebildet ist. Eine ebene Stirnfläche 22.1 liefert eine Anlagefläche für das zweite Teil der drehbaren Verbindung, insbesondere für einen Zahnkranz (nicht dargestellt; vgl. Fig. 7A). Ferner weist die Buchse einen Steg 23 auf, an welchem der Zahnkranz formschlüssig in einer vordefinierten Drehposition relativ zur Buchse 20 positioniert werden kann.

In den Figuren 7A, 7B, 7C und 7D ist ein weiteres Ausführungsbeispiel einer drehbaren Verbindung 1 a in Verbindung mit einem weiteren Ausführungsbeispiel eines anpassbaren Anschlagmechanismus 30b gezeigt. Die drehbare Verbindung 1 a ist an einer Stativvorrichtung 100 vorgesehen, welche ein Tragsystem 101 mit mindestens einem Träger 102 umfasst. Der Anschlagmechanismus 30b weist lediglich einen Stellring 60A auf, welcher mit dem zweiten Teil 50 durch Eingriff in die korrespondierende formschlüssige Kontur 54 zusammenwirkt.

Die einzelnen Komponenten des Anschlagmechanismus 30b, insbesondere der Anschlagring 40 und der Stellring 60A, können auf dieselbe Art zusammenwirken wie im Zusammenhang mit den Figuren 1A bis 3B beschrieben. Der Anschlagmechanismus 30b weist an zwei axial stirnseitigen Schnittstellen jeweils eine formschlüssige Kontur auf und umfasst dabei (nur) einen Stellring 60A.

Der zweite Teil 50 ist nicht in die Buchse 20 integriert. Vielmehr weist der zweite Teil 50 einen Formschlussabschnitt 51 auf, hier in Form einer Gabel. Der Formschlussabschnitt 51 umgreift in radialer Richtung den Steg 23 der Buchse 20 und kann dadurch eine verdrehsichere Anordnung sicherstellen. Wie in Zusammenschau mit der Figur 7B erkennbar, ist eine ebene Unterseite 56, insbesondere eine ringförmige Auflagefläche des zweiten Teils 50 auf einer ebenen Stirnfläche 22.1 der Buchse 20 anordenbar.

In der Figur 7C ist die drehbare Verbindung in einer eingestellten Anordnung gezeigt, wobei der Anschlagring 40 mittels des Formschlusselements 43 in eine Verdrehsicherung bzw. Nut 13 der Spindel 10 eingreift.

In der Figur 7D sind der Anschlagring 40, der Stellring 60A und der Zahnkranz 50 im Querschnitt gezeigt. Der Anschlagring 40 umgreift den Gegenanschlag 62A des Stellrings 60A. Der Gegenanschlag 62A ist in einer Ringkavität 48 des Rings 40 angeordnet. Eine axiale Verlagerung des Stellrings 60A relativ zum Zahnkranz 50 wird durch die Sicherungskappe 95 verhindert, wobei die Sicherungskappe 95 mittels des Rings 40 axial positioniert wird. Der Stellring 60A ist an einem axial hervorstehenden Rand 50.3 des Zahnkranzes 50 zentriert. Der Zahnkranz 50 kann dabei wahlweise an der Spindel oder an der Buchse zentriert werden.

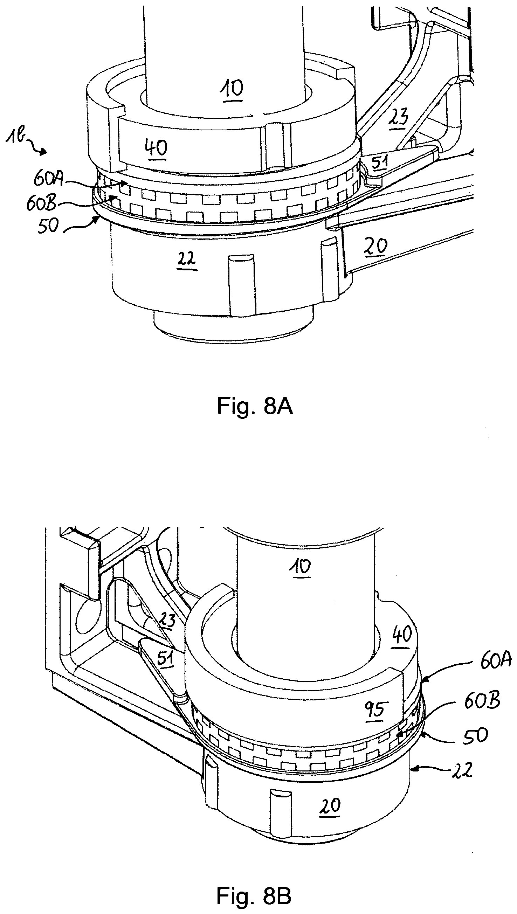

In den Figuren 8A, 8B ist ein weiteres Ausführungsbeispiel einer drehbaren Verbindung 1 b gezeigt, welche den in den Figuren 3A, 3B gezeigten Anschlagmechanismus 30a aufweist, also zwei Stellringe 60A, 60B in Verbindung mit vier axial stirnseitigen Schnittstellen.

### Bezugszeichenliste

- 1, 1a, 1b: drehbare Verbindung
- 10: erstes Verbindungsbauteil, insbesondere Spindel
- 11: Nut
- 12: Ring
- 13: Verdrehsicherung, insbesondere Radialbolzen oder Nut in Außenmantelfläche
- 20: zweite Verbindungsbauteil, insbesondere Buchse
- 21: Durchführung für erstes Verbindungsbauteil
- 22: Ringabschnitt der gabelförmigen Buchse
- 22.1: Stirnfläche
- 22.2: nach innen weisende Mantelfläche
- 23: Steg
- 30, 30a, 30b: anpassbarer Anschlagmechanismus
- 40: erster Teil, insbesondere doppelwandiger Anschlagring
- 42: Anschlag, insbesondere Radialstrebe, Steg oder Schott
- 42.1: Seitenfläche, insbesondere ebene Anschlagfläche
- 43: Formschlusselement
- 44: Außenwand
- 45: Innenwand, insbesondere Zentrierung
- 46: Unterseite
- 46.1: Anlagefläche, insbesondere ringförmige Gleitfläche
- 47: Oberseite
- 48: Ringkavität (rohrförmige Kavität) zwischen den Wänden
- 50: zweiter Teil, insbesondere Zahnkranz
- 50.3: axial hervorstehender Rand
- 51: Formschlussabschnitt, insbesondere Gabel
- 54: formschlüssige Kontur
- 54.1: einzelner Zahn
- 56: ebene Unterseite, insbesondere ringförmige Auflagefläche
- 57: Oberseite
- 60A: (erste) Anschlageinrichtung, insbesondere (oberer) Stellring
- 60A.1: Außenmantelfläche
- 60A.2: Innenmantelfläche
- 62A: Gegenanschlag
- 62A.1: Seitenfläche (Radialflanke), insbesondere ebene Anschlagfläche
- 62A.2: insbesondere konkav gewölbter Innenflächenabschnitt
- 64A: formschlüssige Kontur
- 64A.1: einzelner Zahn
- 66A: Unterseite
- 67A: Stirnfläche bzw. Oberseite, insbesondere ringförmige Gleitfläche
- 60B: weitere (zweite) Anschlageinrichtung, insbesondere (unterer) Stellring
- 60B.1: Außenmantelfläche
- 60B.2: Innenmantelfläche
- 60B.3: axial hervorstehender Rand
- 62B: Gegenanschlag
- 62B.1: Seitenfläche (Radialflanke), insbesondere ebene Anschlagfläche
- 62B.2: insbesondere konvex gewölbter Außenflächenabschnitt
- 64B: erste formschlüssige Kontur, insbesondere an Unterseite
- 64B.1: einzelner Zahn
- 65B: zweite formschlüssige Kontur, insbesondere an Oberseite
- 65B.1: einzelner Zahn
- 66B: Unterseite
- 67B: Stirnfläche bzw. Oberseite
- 70: Zwischenelement
- 74: (erste) formschlüssige Kontur
- 74.1: einzelne Nut
- 75: zweite formschlüssige Kontur
- 75.1: einzelne Nut
- 76: erste Stirnfläche
- 77: zweite Stirnfläche
- 80: Sicherungsring
- 90: Dämpfungselement
- 94: formschlüssige Kontur
- 94.1: einzelner Zahn, insbesondere seitens der Anschlageinrichtung
- 94.2: einzelner Zahn, insbesondere seitens des zweiten Teils
- 95: Sicherungseinrichtung, insbesondere Kappe
- 95.1: (beidseitiger) Innenrand, insbesondere Schenkel eines U-förmigen Profils
- 96: Unterseite
- 96.1: Anlagefläche, insbesondere ringförmige Gleitfläche
- 100: Stativvorrichtung
- 101: Tragsystem
- 102: Träger
- R: Drehachse
- α: Winkel der beiden Stirnflächen des Zwischenelementes zueinander

## Patentansprüche

1. Drehbare Verbindung (1; 1 a; 1 b) für eine Stativvorrichtung (100) zur Anordnung in einem Operationssaal, umfassend einen anpassbaren Anschlagmechanismus (30; 30a; 30b), der zwischen einem ersten Verbindungsbauteil (10) und einem relativ zum ersten Verbindungsbauteil (10) verdrehbar um eine Drehachse (R) gelagerten zweiten Verbindungsbauteil (20) anordenbar ist und eingerichtet ist, mindestens zwei unterschiedliche relative Drehwinkel der Verbindungsbauteile (10, 20) relativ zueinander oder mindestens zwei unterschiedliche Drehbereiche festzulegen, wobei der anpassbare Anschlagmechanismus (30; 30a; 30b) aufweist:
- einen ersten Teil (40), welcher verdrehsicher am ersten Verbindungsbauteil (10) lagerbar ist und einen Anschlag (42) aufweist;
- einen zweiten Teil (50), welcher verdrehsicher am zweiten Verbindungsbauteil (20) anordenbar ist;
wobei der erste Teil (40) relativ zum zweiten Teil (50) verdrehbar gelagert ist;
**dadurch gekennzeichnet, dass** der anpassbare Anschlagmechanismus (30; 30a; 30b) mindestens eine Anschlageinrichtung (60A, 60B) mit jeweils einem Gegenanschlag (62A, 62B) aufweist, welche axial zwischen den beiden Teilen (40, 50) verdrehsicher am zweiten Teil (50) angeordnet ist, wobei der jeweilige Gegenanschlag (62A, 62B) mit dem Anschlag (42) korrespondiert, und wobei die mindestens eine Anschlageinrichtung (60A, 60B) eingerichtet ist, mittels des Gegenanschlags (62A, 62B) die unterschiedlichen Drehwinkel oder Drehbereiche anzupassen.

2. Drehbare Verbindung (1; 1 a; 1 b) nach Anspruch 1, **dadurch gekennzeichnet, dass** der erste Teil (40) eingerichtet ist, den Anschlag (42) mit mindestens jeweils einem Gegenanschlag (62A, 62B) von mindestens zwei Anschlageinrichtungen (60A, 60B) zu kuppeln, insbesondere durch axiales Überlappen, bevorzugt derart, dass der Anschlag entweder mit jedem Gegenanschlag separat in einzelnen Drehwinkelpositionen zusammenwirkt oder gleichzeitig mit mindestens zwei Gegenanschlägen in derselben Drehwinkelposition.

3. Drehbare Verbindung (1; 1 a; 1 b) nach Anspruch 1 oder 2, **dadurch gekennzeichnet, dass** der erste Teil (40) ringförmig ist und eingerichtet ist, den Gegenanschlag (62A, 62B) der mindestens einen Anschlageinrichtung (60A, 60B) in axialer Richtung mittels des Anschlags (42) axial zu überlappen, wobei der erste Teil (40) bevorzugt als doppelwandiger Ring, insbesondere mit U-förmigem Querschnittsprofil, ausgebildet ist, und wobei der Anschlag (42) bevorzugt in Form eines sich in radialer Richtung erstreckenden Stegs ausgebildet ist, welcher bevorzugt eine Innenwand mit einer Außenwand des Rings verbindet.

4. Drehbare Verbindung (1; 1 a; 1 b) nach einem der vorhergehenden Ansprüche, **dadurch gekennzeichnet, dass** der anpassbare Anschlagmechanismus (30; 30a) an vier Schnittstellen, insbesondere axial stirnseitigen Schnittstellen, jeweils eine formschlüssige Kontur (64A; 64B, 65B; 54) aufweist, wobei eine Komponente des anpassbare Anschlagmechanismus (30; 30a; 30b) beidseitig eine formschlüssige Kontur (64B, 65B) aufweist.

5. Drehbare Verbindung (1; 1 a; 1 b) nach einem der vorhergehenden Ansprüche, **dadurch gekennzeichnet, dass** die mindestens eine Anschlageinrichtung (60A, 60B) derart angeordnet ist, dass eine verdrehsichere Anordnung der mindestens einen Anschlageinrichtung (60A, 60B) am zweiten Teil (50) durch eine auf die mindestens eine Anschlageinrichtung (60A, 60B) wirkende Gewichtskraft sichergestellt ist.

6. Drehbare Verbindung (1; 1 a; 1 b) nach einem der vorhergehenden Ansprüche, **dadurch gekennzeichnet, dass** der zweite Teil eine formschlüssige Kontur (54) zum Festlegen einzelner Drehwinkelpositionen aufweist, insbesondere an einer in die axiale Richtung zu der mindestens einen Anschlageinrichtung (60A, 60B) weisenden Oberseite (57), und dass die mindestens eine Anschlageinrichtung (60A, 60B) eine damit korrespondierende formschlüssige Kontur (64A) aufweist, insbesondere an einer in axialer Richtung zum zweiten Teil (50) hin weisenden Unterseite (66A, 66B).

7. Drehbare Verbindung (1; 1 a; 1 b) nach einem der vorhergehenden Ansprüche, **dadurch gekennzeichnet, dass** der anpassbare Anschlagmechanismus (30; 30a; 30b) mindestens zwei Anschlageinrichtungen (60A, 60B) aufweist, welche axial hintereinander angeordnet sind, wobei jede Anschlageinrichtung (60A, 60B) einen in axialer Richtung hervorstehenden Gegenanschlag (62A, 62B) aufweist, und wobei die Gegenanschläge bevorzugt in axialer Richtung überlappend anordenbar sind, wobei bevorzugt eine der Anschlageinrichtungen (60A) auf einer Unterseite (66A) eine formschlüssige Kontur (64A) aufweist, und die andere der Anschlageinrichtungen (60B) auf einer Oberseite (67B) und einer Unterseite (66B) jeweils eine formschlüssige Kontur (64B, 65B) aufweist, wobei die formschlüssigen Konturen (64A, 64B, 65B) bevorzugt jeweils als eine Vielzahl symmetrisch umlaufend angeordneter, radial ausgerichteter Zähne (64A.1, 64B.1, 65B.1) ausgebildet sind.

8. Drehbare Verbindung (1; 1 a; 1 b) nach Anspruch 7, **dadurch gekennzeichnet, dass** sowohl die formschlüssige Kontur der einen Anschlageinrichtung (60A) als auch eine der formschlüssigen Konturen der anderen Anschlageinrichtung (60B) geometrisch korrespondierend zu einer/der formschlüssigen Kontur des zweiten Teils ausgebildet ist.

9. Drehbare Verbindung (1; 1 a; 1 b) nach einem der vorhergehenden Ansprüche, **dadurch gekennzeichnet, dass** der anpassbare Anschlagmechanismus (30; 30a; 30b) eine Sicherungseinrichtung (95) aufweist, insbesondere eine bevorzugt halbschalenförmige Kappe, welche am ersten Teil anordenbar ist und eingerichtet ist, mit der mindestens einen Anschlageinrichtung zusammenzuwirken, insbesondere eine axiale Verlagerung der mindestens einen Anschlageinrichtung relativ zum ersten Teil zu verhindern.

10. Drehbare Verbindung (1; 1 a; 1 b) nach einem der vorhergehenden Ansprüche, **dadurch gekennzeichnet, dass** der anpassbare Anschlagmechanismus (30; 30a; 30b) ein Dämpfungselement, insbesondere aus Elastomermaterial, aufweist, welches mit dem zweiten Teil und/oder der mindestens einen Anschlageinrichtung korrespondiert, insbesondere mit einer stirnseitig in axialer Richtung hervorstehenden formschlüssigen Kontur (64A) der jeweiligen Anschlageinrichtung.

11. Drehbare Verbindung (1; 1 a; 1 b) nach einem der vorhergehenden Ansprüche, **dadurch gekennzeichnet, dass** die drehbare Verbindung ein Zwischenelement (70) aufweist, welches axial zwischen dem ersten Teil (40), insbesondere zwischen der mindestens einen Anschlageinrichtung (60A, 60B), und dem zweiten Verbindungsbauteil (20) angeordnet ist und mindestens eine formschlüssige Kontur (74, 75) zur drehfesten Verbindung mit der mindestens einen Anschlageinrichtung (60A, 60B) oder dem zweiten Verbindungsbauteil (20) aufweist, wobei bevorzugt an zwei gegenüberliegenden Stirnflächen (76, 77) des Zwischenelements jeweils eine formschlüssige Kontur (74, 75) angeordnet ist.

12. Tragsystem für eine Stativvorrichtung (100) zur Anordnung in einem Operationssaal und zum Positionieren einer medizintechnischen Einrichtung im Operationssaal, umfassend eine drehbare Verbindung (1; 1 a; 1 b) nach einem der Ansprüche 1 bis 13 sowie das erste Verbindungsbauteil (10), insbesondere in Form einer Spindel, und das zweite Verbindungsbauteil (20), insbesondere in Form einer Buchse, wobei das zweite Verbindungsbauteil bevorzugt als gabelförmige Buchse ausgebildet ist, wobei die mindestens eine Anschlageinrichtung (60A, 60B) und der erste Teil (40) und der zweite Teil (50) bevorzugt zwischen zwei Ringabschnitten (22) der Buchse an einem der beiden Ringabschnitte (22) angeordnet sind, und wobei der zweite Teil (50) bevorzugt einen Formschlussabschnitt (51) aufweist, insbesondere eine Gabel, mittels welchem der zweite Teil verdrehsicher am zweiten Verbindungsbauteil, insbesondere an einem Steg (23), positionierbar ist.

13. Stativvorrichtung (100) zur Anordnung in einem Operationssaal und zum Positionieren einer medizintechnischen Einrichtung im Operationssaal, umfassend eine drehbare Verbindung (1; 1a; 1b) nach einem der Ansprüche 1 bis 11 oder ein Tragsystem nach Anspruch 12.

14. Verfahren zum Einstellen eines anpassbaren Anschlagmechanismus (30; 30a; 30b) einer drehbaren Verbindung zwischen zwei Verbindungsbauteilen (10, 20), insbesondere einer drehbaren Verbindung (1; 1 a; 1 b) nach einem der Ansprüche 1 bis 13, für eine Stativvorrichtung (100) zur Anordnung in einem Operationssaal, wobei der Anschlagmechanismus einen ersten Teil (40), einen zweiten Teil (50) und mindestens eine Anschlageinrichtung (60A, 60B) mit jeweils einem Gegenanschlag (62A, 62B) aufweist, wobei der zweite Teil (50) verdrehsicher an einem/dem verdrehbar um eine Drehachse (R) gelagerten zweiten Verbindungsbauteil (20) gelagert ist, **gekennzeichnet durch** die Schritte:
- Lösen eines formschlüssigen Eingriffs zwischen der mindestens einen Anschlageinrichtung (60A, 60B) und dem zweiten Teil (50) **durch** axiales Verlagern des ersten Teils (40) und der mindestens einen Anschlageinrichtung (60A, 60B) entlang der Drehachse (R) und entlang des ersten Verbindungsbauteils (10); und
- Festlegen eines Drehbereichs oder eines relativen Drehwinkels der Verbindungsbauteile (10, 20) relativ zueinander **durch** axiales Zurückverlagern des ersten Teils (40) zusammen mit der mindestens einen Anschlageinrichtung (60A, 60B) und formschlüssiges Eingreifen der mindestens einen Anschlageinrichtung (60A, 60B) im zweiten Teil (50) in einer geänderten Drehwinkelposition.

15. Verfahren nach Anspruch 14, wobei der Anschlagmechanismus mindestens zwei Anschlageinrichtungen aufweist, ferner umfassend die Schritte:
- Lösen eines formschlüssigen Eingriffs zwischen dem ersten Teil (40), insbesondere einem Anschlag (42) des ersten Teils (40), und einem/dem jeweiligen Gegenanschlag der mindestens zwei Anschlageinrichtungen (60A, 60B) durch axiales Verlagern des ersten Teils (40) relativ zu den mindestens zwei Anschlageinrichtungen (60A, 60B), insbesondere in Richtung der Drehachse (R);
- Lösen eines formschlüssigen Eingriffs zwischen den mindestens zwei Anschlageinrichtungen (60A, 60B) durch axiales Verlagern der Anschlageinrichtungen (60A, 60B) relativ zueinander; und
- Verdrehen der Anschlageinrichtungen (60A, 60B), insbesondere der Gegenanschläge, relativ zueinander und dann formschlüssiges Eingreifen der Anschlageinrichtungen (60A, 60B) durch axiales Zurückverlagern der Anschlageinrichtungen (60A, 60B) relativ zueinander, insbesondere durch Ineinanderstecken, in einer geänderten Drehwinkelposition, bevor zumindest eine der Anschlageinrichtungen (60A, 60B) wieder in formschlüssigen Eingriff mit dem zweiten Teil (50) gebracht wird.

## Claims

1. A rotatable connection (1; 1 a; 1 b) for a mounting device (100) for arrangement in an operating room, comprising an adjustable stop mechanism (30; 30a; 30b), which can be disposed between a first connection component (10) and a second connection component (20), which is rotatably mounted about a rotational axis (R) relative to the first connection component (10), and configured to define at least two different relative rotational angles of the connection components (10, 20) relative to each other or at least two different ranges of rotation, the adjustable stop mechanism (30; 30a; 30b) comprising:
- a first part (40), which can be mounted on the first connection component (10) in a non-rotatable manner and comprises a stop (41); and
- a second part (50), which can be disposed on the second connection component (20) in a torsion-proof manner;
the first part (40) being rotatably mounted relative to the second part (50);
**characterized in that** the adjustable stop mechanism (30; 30a; 30b) comprises at least one stop device (60A, 60B) which has a respective counterstop (62A, 62B) and is disposed in a torsion-proof manner on the second part (50) axially between the two parts (40, 50), the respective counterstop (62A, 62B) corresponding to the stop (42), and the at least one stop device (60A, 60B) being configured to adjust the different rotational angles or ranges of rotation by way of the counterstop (62A, 62B).

2. The rotatable connection (1; 1a; 1b) according to claim 1, **characterized in that** the first part (40) is configured to couple the stop (42) to at least one respective counterstop (62A, 62B) of at least two stop devices (60A, 60B), in particular by axial overlapping, preferably in such a way that the stop either separately cooperates with each counterstop in individual rotational angle positions, or simultaneously with at least two counterstops in the same rotational angle position.

3. The rotatable connection (1; 1a; 1b) according to claim 1 or 2, **characterized in that** the first part (40) is annular and configured to axially overlap the counterstop (62A, 62B) of the at least one stop device (60A, 60B) in the axial direction by way of the stop (42), the first part (40) preferably being designed as a double-walled ring, in particular having a U-shaped cross-sectional profile, and the stop (42) preferably being designed in the form of a rib extending in the radial direction, which preferably connects an inner wall to an outer wall of the ring.

4. A rotatable connection (1; 1a; 1b) according to any one of the preceding claims, **characterized in that** the adjustable stop mechanism (30; 30a) has a respective formtocked contour (64A; 64B; 65B; 54) on each of four interfaces, in particular interfaces located at axial end faces, a component of the adjustable stop mechanism (30; 30a; 30b) having a form-locked contour (64B, 65B) on both sides.

5. A rotatable connection (1; 1a; 1 b) according to any one of the preceding claims, **characterized in that** the at least one stop device (60A, 60B) is disposed in such a way that a torsion-proof arrangement of the at least one stop device (60A, 60B) on the second part is ensured by a weight acting on the at least one stop device (60A, 60B).

6. A rotatable connection (1; 1a; 1 b) according to any one of the preceding claims, **characterized in that** the second part has a form-locked contour (54) for defining individual rotational angle positions, in particular on an upper face (57) pointing in the axial direction toward the at least one stop device (60A, 60B), and the at least one stop device (60A, 60B) has a form-locked contour (64A) corresponding thereto, in particular on a lower face (66A, 66B) pointing in the axial direction toward the second part (50).

7. A rotatable connection (1; 1 a; 1 b) according to any one of the preceding claims,
**characterized in that** the adjustable stop mechanism (30; 30a; 30b) comprises at least two stop devices (60A, 60B), which are disposed axially behind each other, each stop device (60A, 60B) comprising a counterstop (62A, 62B) protruding in the axial direction, and the counterstops preferably being disposable in an overlapping manner in the axial direction, preferably one of the stop devices (60A) having a form-locked contour (64A) on a lower face (66A), and the other of the stop devices (60B) having a respective form-locked contour (64B, 65B) on an upper face (67B) and a lower face (66B), the form-locked contours (64A, 64B, 65B) preferably being designed in each case as a plurality of symmetrically circumferentially disposed, radially oriented teeth (64A.1, 64B.1, 65B.1).

8. The rotatable connection (1; 1a; 1 b) according to claim 7, **characterized in that** both the form-locked contour of the one stop device (60A) and one of the form-locked contours of the other stop device (60B) are formed so as to geometrically correspond to a/the form-locked contour of the second part.

9. A rotatable connection (1; 1 a; 1 b) according to any one of the preceding claims, **characterized in that** the adjustable stop mechanism (30; 30a; 30b) comprises a retaining device (95), in particular a preferably half shell-shaped cap, which can be disposed on the first part and is configured to cooperate with the at least one stop device, in particular to prevent an axial displacement of the at least one stop device relative to the first part.

10. A rotatable connection (1; 1 a; 1 b) according to any one of the preceding claims, **characterized in that** the adjustable stop mechanism (30; 30a; 30b) comprises a damping element, in particular made of elastomer material, which corresponds to the second part and/or to the at least one stop device, in particular having a form-locked contour (64A) of the respective stop device protruding at the end face in the axial direction.

11. A rotatable connection (1; 1 a; 1 b) according to any one of the preceding claims, **characterized in that** the rotatable connection comprises an intermediate element (70), which is disposed axially between the first part (40), in particular between the at least one stop device (60A, 60B), and the second connection part (20) and has at least one form-locked contour (74, 75) for the non-rotatable connection to the at least one stop device (60A, 60B) or the second connection component (20), preferably a respective form-locked contour (74, 75) being provided on each of the two opposing end faces (76, 77) of the intermediate element.

12. A support system for a mounting device (100) for arrangement in an operating room and for positioning a piece of medical equipment in the operating room, comprising a rotatable connection (1; 1 a; 1 b) according to any one of claims 1 to 13, and the first connection component (10), in particular in the form of a spindle, and the second connection component (20), in particular in the form of a sleeve, the second connection component preferably being designed as a fork-shaped sleeve, the at least one stop device (60A, 60B) and the first part (40) and the second part (50) preferably being disposed between two ring sections (22) of the sleeve on one of the two ring sections (22), and the second part (50) preferably having a form-locked section (51), in particular a fork, by way of which the second part can be positioned in a torsion-proof manner on the second connection component, in particular on a rib (23).

13. The mounting device (100) for arrangement in an operating room and for positioning a piece of medical equipment in the operating room, comprising a rotatable connection (1; 1 a; 1 b) according to any one of claims 1 to 11 or a support system according to claim 12.

14. A method for setting an adjustable stop mechanism (30; 30a; 30b) of a rotatable connection between two connection components (10, 20), in particular of a rotatable connection (1; 1a; 1b) according to any one of claims 1 to 13, for a mounting device (100) for arrangement in an operating room, the stop mechanism comprising a first part (40), a second part (50) and at least one stop device (60A, 60B) having a respective counterstop (62A, 62B), the second part (50) being mounted in a torsion-proof manner on a/the second connection component (20), which is mounted rotatably about a rotational axis (R), **characterized by** the following steps:
- releasing a form-locked engagement between the at least one stop device (60A, 60B) and the second part (50) by axial displacement of the first part (40) and of the at least one stop device (60A, 60B) along the rotational axis (R) and along the first connection component (10); and
- defining a range of rotation or a relative rotational angle of the connection components (10, 20) relative to each other by axial re-displacement of the first part (40), together with the at least one stop device (60A, 60B), and form-locked engagement of the at least one stop device (60A, 60B) in the second part (50) in a changed rotational angle position.

15. The method according to claim 14, wherein the stop mechanism comprises at least two stop devices, further comprising the following steps:
- releasing a form-locked engagement between the first part (40), in particular a stop (42) of the first part (40), and a/the respective counterstop of the at least two stop devices (60A, 60B) by axial displacement of the first part (40) relative to the at least two stop devices (60A, 60B), in particular in the direction of the rotational axis (R);
- releasing a form-locked engagement between the at least two stop devices (60A, 60B) by axial displacement of the stop devices (60A, 60B) relative to each other; and
- rotating the stop devices (60A, 60B), in particular the counterstops, relative to each other and then engaging, in a form-locked manner, the stop devices (60A, 60B) by axial re-displacement of the stop devices (60A, 60B) relative to each other, in particular by plugging them into each other, in a changed rotational angle position before at least one of the stop devices (60A, 60B) is again brought into form-locked engagement with the second part (50).

## Revendications

1. Liaison rotative (1 ; 1a ; 1b) pour un dispositif à trépied (100) pour la disposition dans une salle d'opération, comprenant un mécanisme de butée adaptable (30 ; 30a ; 30b), qui peut être disposé entre un premier composant de liaison (10) et un deuxième composant de liaison (20) logé de manière rotative par rapport au premier composant de liaison (10) autour d'un axe de rotation (R) et qui est conçu pour fixer au moins deux angles de rotation relatifs différents des composants de liaison (10, 20) entre eux ou au moins deux zones angulaires différentes, le mécanisme de butée adaptable (30 ; 30a ; 30b) comprenant :
- une première partie (40) qui peut être logée de manière solidaire en rotation sur un premier composant de liaison (10) et qui comprend une butée (42) ;
- une deuxième partie (50) qui peut être disposée de manière solidaire en rotation sur un deuxième composant de liaison (20) ;
la première partie (40) étant logée de manière rotative par rapport à la deuxième partie (50) ;
**caractérisée en ce que** le mécanisme de butée adaptable (30 ; 30a ; 30b) comprend au moins un dispositif de butée (60A, 60B), avec une contre-butée (62A, 62B), qui est disposé axialement entre les deux parties (40, 50) de manière solidaire en rotation sur la deuxième partie (50), la contre-butée (62A, 62B) correspondant à la butée (42) et l'au moins un dispositif de butée (60A, 60B) étant conçu pour adapter, au moyen de la contre-butée (62A, 62B), des différents angles de rotation ou zones de rotation.

2. Liaison rotative (1 ; 1a ; 1b) selon la revendication 1, **caractérisée en ce que** la première partie (40) est conçue pour coupler la butée (42) avec au moins une contre-butée (62A, 62B) d'au moins deux dispositifs de butée (60A, 60B), plus particulièrement par superposition axiale, de préférence de façon à ce que la butée interagisse soit séparément avec chaque contre-butée dans différentes positions angulaires de rotation soit simultanément avec au moins deux contre-butées dans la même position angulaire de rotation.

3. Liaison rotative (1 ; la ; 1b) selon la revendication 1 ou 2, **caractérisée en ce que** la première partie (40) présente une forme annulaire et est conçue pour se superposer axialement avec la contre-butée (62A, 62B) de l'au moins un dispositif de butée (60A, 60B) dans la direction axiale au moyen de la butée (42), la première partie (40) étant conçue de préférence sous la forme d'un anneau à double paroi, plus particulièrement avec un profil de section transversale en forme de U et la butée (42) se présentant de préférence sous la forme d'une nervure s'étendant dans la direction radiale, qui relie de préférence une paroi interne avec une paroi externe de l'anneau.

4. Liaison rotative (1 ; 1a ; 1b) selon l'une des revendications précédentes, **caractérisée en ce que** le mécanisme de butée adaptable (30 ; 30a) comprend, au niveau de chacun de quatre interfaces, plus particulièrement d'interfaces frontales axiales, un contour à complémentarité de forme (64A ; 64B, 65B ; 54), un composant du mécanisme de butée adaptable (30 ; 30a ; 30b) présentant des deux côtés un contour de forme complémentaire (64B, 65B).

5. Liaison rotative (1 ; 1a ; 1b) selon l'une des revendications précédentes, **caractérisée en ce que** l'au moins un dispositif de butée (60A, 60B) est disposé de façon à ce qu'une disposition solidaire en rotation de l'au moins un dispositif de butée (60A, 60B) sur la deuxième partie (50) est garantie par une force de gravité agissant sur l'au moins un dispositif de butée (60A, 60B).

6. Liaison rotative (1 ; 1a ; 1b) selon l'une des revendications précédentes, **caractérisée en ce que** la deuxième partie présente un contour à complémentarité de forme (54) pour la fixation de différentes positions angulaires de rotation, plus particulièrement au niveau d'un côté supérieur (57) orienté dans la direction axiale vers l'au moins un dispositif de butée (60A, 60B) et **en ce que** l'au moins un dispositif de butée (60A, 60B) présente un contour à complémentarité de forme (64A) correspondant, plus particulièrement au niveau d'un côté inférieur (66A, 66B) orienté dans la direction axiale vers la deuxième partie (50).

7. Liaison rotative (1 ; 1a ; 1b) selon l'une des revendications précédentes, **caractérisée en ce que** le mécanisme de butée adaptable (30 ; 30a ; 30b) comprend au moins deux dispositifs de butée (60A, 60B) qui sont disposés axialement l'un derrière l'autre, chaque dispositif de butée (60A, 60B) comprenant une contre-butée (62A, 62B) dépassant dans la direction axiale et les contre-butées pouvant être disposées de façon à se superposer dans la direction axiale, de préférence un des dispositifs de butée (60A) comprenant, sur un côté inférieur (66A), un contour à complémentarité de forme (64A) et l'autre dispositif de butée (60B) comprenant, sur un côté supérieur (67B) et un côté inférieur (66B), respectivement un contour à complémentarité de forme (64B, 65B), les contours à complémentarité de forme (64A, 64B, 65B) étant de préférence conçus sous la forme d'une pluralité de dents (64A.1, 64B.1, 65B.1) disposées de manière circulaire et symétrique et orientées radialement.

8. Liaison rotative (1 ; 1a ; 1b) selon la revendication 7, **caractérisée en ce que** le contour à complémentarité de forme d'un dispositif de butée (60A) ainsi qu'un des contours à complémentarité de forme de l'autre dispositif de butée (60B) présentent une forme géométrique correspondant à un/le contour à complémentarité de forme de la deuxième partie.

9. Liaison rotative (1 ; 1a ; 1b) selon l'une des revendications précédentes, **caractérisée en ce que** le mécanisme de butée adaptable (30 ; 30a, 30b) comprend un dispositif de fixation (95), plus particulièrement un capuchon de préférence en forme de demi-coque, qui peut être disposé sur la première partie et qui est conçu pur interagir avec l'au moins un dispositif de butée, plus particulièrement pour empêcher un déplacement axial de l'au moins un dispositif de butée par rapport à la première partie.

10. Liaison rotative (1 ; 1a ; 1b) selon l'une des revendications précédentes, **caractérisée en ce que** le mécanisme de butée adaptable (30 ; 30a, 30b) comprend un élément d'amortissement, plus particulièrement constitué d'un matériau élastomère, qui correspond à la deuxième partie et/ou à l'au moins un dispositif de butée, plus particulièrement avec un contour à complémentarité de forme (64A), dépassant frontalement dans la direction axiale, du dispositif de butée correspondant.

11. Liaison rotative (1 ; 1a ; 1b) selon l'une des revendications précédentes, **caractérisée en ce que** la liaison rotative comprend un élément intermédiaire (70) qui est disposé axialement entre la première partie (40), plus particulièrement entre l'au moins un dispositif de butée (60A, 60B) et le deuxième composant de liaison (20), et au moins un contour à complémentarité de forme (74, 75) pour la liaison solidaire en rotation avec l'au moins un dispositif de butée (60A, 60B) ou le deuxième composant de liaison (20), un contour à complémentarité de forme (74, 75) étant disposé de préférence respectivement au niveau de deux faces frontales opposées (76, 77) de l'élément intermédiaire.

12. Système de support pour un dispositif à trépied (100) pour la disposition dans une salle d'opération et pour le positionnement d'un dispositif médical technique dans la salle d'opération, comprenant une liaison rotative (1 ; 1a ; 1b) selon l'une des revendications 1 à 13, ainsi que le premier composant de liaison (10), plus particulièrement sous la forme d'une broche, et le deuxième composant de liaison (20), plus particulièrement sous la forme d'une douille, le deuxième composant de liaison étant conçu de préférence comme une douille en forme de fourche, l'au moins un dispositif de butée (60A, 60B) et la première partie (40) et la deuxième partie (50) étant disposés de préférence entre deux portions annulaires (22) de la douille au niveau d'une des deux portions annulaires (22) et la deuxième partie (50) présentant de préférence une portion à complémentarité de forme (51), plus particulièrement une fourche, à l'aide de laquelle la deuxième partie peut être positionnée de manière solidaire en rotation sur le deuxième composant de liaison, plus particulièrement au niveau d'une nervure (23).

13. Dispositif à trépied (100) pour la disposition dans une salle d'opération et pour le positionnement d'un dispositif médical technique dans la salle d'opération, comprenant une liaison rotative (1 ; 1a ; 1b) selon l'une des revendications 1 à 11 ou un système de support selon la revendication 12.

14. Procédé de réglage d'un mécanisme de butée adaptable (30 ; 30a ; 30b) d'une liaison rotative entre deux composants de liaison (10, 20), plus particulièrement d'une liaison rotative (1 ; la ; 1b) selon l'une des revendications 1 à 13, pour un dispositif à trépied (100) pour la disposition dans une salle d'opération, le mécanisme de butée comprenant une première partie (40), une deuxième partie (50) et au moins un dispositif de butée (60A, 60B) avec une contre-butée (62A, 62B), la deuxième partie (50) étant logée de manière solidaire en rotation au niveau d'un/du deuxième composant de liaison (20) logé de manière rotative autour d'un axe de rotation (R), **caractérisé par** les étapes suivantes :
- déverrouillage d'un emboîtement à complémentarité de forme entre l'au moins un dispositif de butée (60A, 60B) et la deuxième partie (50) par déplacement axial de la première partie (40) et de l'au moins un dispositif de butée (60A, 60B) le long de l'axe de rotation (R) et le long du premier composant de liaison (10) ; et
- détermination d'une zone angulaire ou d'un angle de rotation relatif des composants de liaison (10, 20) l'un par rapport à l'autre par retour axial de la première partie (40) simultanément avec l'au moins un dispositif de butée (60A, 60B) et emboîtement à complémentarité de forme de l'au moins un dispositif de butée (60A, 60B) dans la deuxième partie (50) dans une position angulaire de rotation modifiée.

15. Procédé selon la revendication 14, le mécanisme de butée comprenant au moins deux dispositifs de butée, comprenant en outre les étapes suivantes :
- déverrouillage d'un emboîtement à complémentarité de forme entre la première partie (40), plus particulièrement une butée (42) de la première partie (40) et une/la contre-butée correspondante des au moins deux dispositifs de butée (60A, 60B) par déplacement axial de la première partie (40) par rapport aux au moins deux dispositifs de butée (60A, 60B), plus particulièrement dans la direction de l'axe de rotation (R) ;
- déverrouillage d'un emboîtement à complémentarité de forme entre les au moins deux dispositifs de butée (60A, 60B) par déplacement axial des dispositifs de butée (60A, 60B) entre eux ; et
- rotation des dispositifs de butée (60A, 60B), plus particulièrement des contre-butées, entre eux, puis emboîtement par complémentarité de forme des dispositifs de butée (60A, 60B) par retour axial des dispositifs de butée (60A, 60B) entre eux, plus particulièrement par emboîtement mutuel, dans une position angulaire de rotation modifiée, avant qu'au moins un des dispositifs de butée (60A, 60B) soit à nouveau emboîté par complémentarité de forme avec la deuxième partie (50).
